(19) **Europäisches Patentamt European Patent Office Office européen des brevets**

(11) **EP 4 061 458 B1**

(12) **EUROPEAN PATENT SPECIFICATION**

(45) Date of publication and mention of the grant of the patent:
**08.10.2025 Bulletin 2025/41**

(21) Application number: **20890114.0**

(22) Date of filing: **15.10.2020**

(51) International Patent Classification (IPC):
**A61M 16/00** (2006.01)

(52) Cooperative Patent Classification (CPC):
(C-Sets available)
**G06F 3/017; A61M 16/024; G06V 40/28;**
A61M 16/0051; A61M 16/0069; A61M 16/06;
A61M 16/0683; A61M 16/0875; A61M 16/1005;
A61M 16/109; A61M 16/1095; A61M 16/125;
A61M 16/161; A61M 16/208; A61M 2016/0027;
(Cont.)

(86) International application number:
**PCT/AU2020/051111**

(87) International publication number:
**WO 2021/097517 (27.05.2021 Gazette 2021/21)**

(54) **APPARATUS AND METHOD FOR DETECTING USER INTERACTION WITH A RESPIRATORY THERAPY DEVICE**

VORRICHTUNG UND VERFAHREN ZUR ERKENNUNG EINER BENUTZERINTERAKTION MIT EINER ATEMTHERAPIEVORRICHTUNG

APPAREIL ET PROCÉDÉ DE DÉTECTION D'UNE INTERACTION D'UTILISATEUR AVEC UN DISPOSITIF DE THÉRAPIE RESPIRATOIRE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **22.11.2019 AU 2019904413**

(43) Date of publication of application:
**28.09.2022 Bulletin 2022/39**

(73) Proprietor: **ResMed Pty Ltd**
**Bella Vista, New South Wales 2153 (AU)**

(72) Inventors:
• **RICHARDS, David**
**Bella Vista, New South Wales 2153 (AU)**
• **HART, Stephanie**
**Bella Vista, New South Wales 2153 (AU)**
• **DONKER, Clare**
**Bella Vista, New South Wales 2153 (AU)**

(74) Representative: **Vossius & Partner**
**Patentanwälte Rechtsanwälte mbB**
**Siebertstraße 3**
**81675 München (DE)**

(56) References cited:
WO-A1-2015/089582    WO-A1-2018/032042
US-A1- 2002 179 088    US-A1- 2007 193 582
US-A1- 2007 193 582    US-A1- 2019 187 645
US-B1- 6 240 921

(52) Cooperative Patent Classification (CPC): (Cont.)
A61M 2016/0039; A61M 2205/121; A61M 2205/123;
A61M 2205/127; A61M 2205/13; A61M 2205/14;
A61M 2205/18; A61M 2205/276; A61M 2205/3306;
A61M 2205/3317; A61M 2205/332;
A61M 2205/3331; A61M 2205/3368;
A61M 2205/3375; A61M 2205/3389;

A61M 2205/3592; A61M 2205/42; A61M 2205/502;
A61M 2205/505; A61M 2205/581; A61M 2205/582;
A61M 2205/583; A61M 2205/8212; A61M 2230/63;
G06N 20/00

C-Sets
A61M 2230/63, A61M 2230/005

**Description**

1 CROSS-REFERENCE TO RELATED APPLICATIONS

This application claims the benefit of Australian Provisional Application No. 2019904413 filed November 22 2019.

2 BACKGROUND OF THE TECHNOLOGY

2.1 FIELD OF THE TECHNOLOGY

The present technology relates to one or more of the screening, diagnosis, monitoring, treatment, prevention and amelioration of respiratory-related disorders. The present technology also relates to medical devices or apparatus, and their use. The present technology also relates to methods of detecting user interaction with respiratory therapy devices.

2.2 DESCRIPTION OF THE RELATED ART

**2.2.1 Human Respiratory** System **and its Disorders**

The respiratory system of the body facilitates gas exchange. The nose and mouth form the entrance to the airways of a patient.

The airways include a series of branching tubes, which become narrower, shorter and more numerous as they penetrate deeper into the lung. The prime function of the lung is gas exchange, allowing oxygen to move from the inhaled air into the venous blood and carbon dioxide to move in the opposite direction. The trachea divides into right and left main bronchi, which further divide eventually into terminal bronchioles. The bronchi make up the conducting airways, and do not take part in gas exchange. Further divisions of the airways lead to the respiratory bronchioles, and eventually to the alveoli. The alveolated region of the lung is where the gas exchange takes place, and is referred to as the respiratory zone. See *"Respiratory Physiology"*, by John B. West, Lippincott Williams & Wilkins, 9th edition published 2012.

A range of respiratory disorders exist. Certain disorders may be characterised by particular events, e.g. apneas, hypopneas, and hyperpneas.

Examples of respiratory disorders include Obstructive Sleep Apnea (OSA), Cheyne-Stokes Respiration (CSR), respiratory insufficiency, Obesity Hyperventilation Syndrome (OHS), Chronic Obstructive Pulmonary Disease (COPD), Neuromuscular Disease (NMD) and Chest wall disorders.

Obstructive Sleep Apnea (OSA), a form of Sleep Disordered Breathing (SDB), is characterised by events including occlusion or obstruction of the upper air passage during sleep. It results from a combination of an abnormally small upper airway and the normal loss of muscle tone in the region of the tongue, soft palate and posterior oropharyngeal wall during sleep. The condition causes the affected patient to stop breathing for periods typically of 30 to 120 seconds in duration, sometimes 200 to 300 times per night. It often causes excessive daytime somnolence, and it may cause cardiovascular disease and brain damage. The syndrome is a common disorder, particularly in middle aged overweight males, although a person affected may have no awareness of the problem. See US Patent No. 4,944,310 (Sullivan).

Cheyne-Stokes Respiration (CSR) is another form of sleep disordered breathing. CSR is a disorder of a patient's respiratory controller in which there are rhythmic alternating periods of waxing and waning ventilation known as CSR cycles. CSR is characterised by repetitive de-oxygenation and re-oxygenation of the arterial blood. It is possible that CSR is harmful because of the repetitive hypoxia. In some patients CSR is associated with repetitive arousal from sleep, which causes severe sleep disruption, increased sympathetic activity, and increased afterload. See US Patent No. 6,532,959 (Berthon-Jones).

Respiratory failure is an umbrella term for respiratory disorders in which the lungs are unable to inspire sufficient oxygen or exhale sufficient $CO_2$ to meet the patient's needs. Respiratory failure may encompass some or all of the following disorders.

A patient with respiratory insufficiency (a form of respiratory failure) may experience abnormal shortness of breath on exercise.

Obesity Hyperventilation Syndrome (OHS) is defined as the combination of severe obesity and awake chronic hypercapnia, in the absence of other known causes for hypoventilation. Symptoms include dyspnea, morning headache and excessive daytime sleepiness.

Chronic Obstructive Pulmonary Disease (COPD) encompasses any of a group of lower airway diseases that have certain characteristics in common. These include increased resistance to air movement, extended expiratory phase of respiration, and loss of the normal elasticity of the lung. Examples of COPD are emphysema and chronic bronchitis. COPD is caused by chronic tobacco smoking (primary risk factor), occupational exposures, air pollution and genetic factors. Symptoms include: dyspnea on exertion, chronic cough and sputum production.

Neuromuscular Disease (NMD) is a broad term that encompasses many diseases and ailments that impair the functioning of the muscles either directly via intrinsic muscle pathology, or indirectly via nerve pathology. Some NMD patients are characterised by progressive muscular impairment leading to loss of ambulation, being wheelchair-bound, swallowing difficulties, respiratory muscle weakness and, eventually, death from respiratory failure. Neuromuscular disorders can be divided into rapidly progressive and slowly progressive: (i) Rapidly progressive disorders: Characterised by muscle impairment that worsens over months and results in death within a few years (e.g. Amyotrophic lateral sclerosis (ALS) and Duchenne muscular dystrophy (DMD) in teenagers); (ii) Variable or slowly progressive disorders: Characterised by muscle impairment that worsens over years and only mildly reduces life expectancy (e.g. Limb girdle, Facioscapulohumeral and Myotonic muscular dystrophy). Symptoms of respiratory failure in NMD include: increasing generalised weakness, dysphagia, dyspnea on exertion and at rest, fatigue, sleepiness, morning headache, and difficulties with concentration and mood changes.

Chest wall disorders are a group of thoracic deformities that result in inefficient coupling between the respiratory muscles and the thoracic cage. The disorders are usually characterised by a restrictive defect and share the potential of long term hypercapnic respiratory failure. Scoliosis and/or kyphoscoliosis may cause severe respiratory failure. Symptoms of respiratory failure include: dyspnea on exertion, peripheral oedema, orthopnea, repeated chest infections, morning headaches, fatigue, poor sleep quality and loss of appetite.

A range of therapies have been used to treat or ameliorate such conditions. Furthermore, otherwise healthy individuals may take advantage of such therapies to prevent respiratory disorders from arising. However, these have a number of shortcomings.

### 2.2.2 Therapies

Various respiratory therapies, such as Continuous Positive Airway Pressure (CPAP) therapy, Non-invasive ventilation (NIV), Invasive ventilation (IV), High Flow Therapy (HFT) and long-term oxygen therapy (LTOT) have been used to treat one or more of the above respiratory disorders.

### 2.2.2.1 Respiratory pressure therapies

Respiratory pressure therapy is the application of a supply of air to an entrance to the airways at a controlled target pressure that is nominally positive with respect to atmosphere throughout the patient's breathing cycle (in contrast to negative pressure therapies such as the tank ventilator or cuirass).

Continuous Positive Airway Pressure (CPAP) therapy has been used to treat Obstructive Sleep Apnea (OSA). The mechanism of action is that continuous positive airway pressure acts as a pneumatic splint and may prevent upper airway occlusion, such as by pushing the soft palate and tongue forward and away from the posterior oropharyngeal wall. Treatment of OSA by CPAP therapy may be voluntary, and hence patients may elect not to comply with therapy if they find devices used to provide such therapy one or more of: uncomfortable, difficult to use, expensive and aesthetically unappealing.

Non-invasive ventilation (NIV) provides ventilatory support to a patient through the upper airways to assist the patient breathing and/or maintain adequate oxygen levels in the body by doing some or all of the work of breathing. The ventilatory support is provided via a non-invasive patient interface. NIV has been used to treat CSR and respiratory failure, in forms such as OHS, COPD, NMD and Chest Wall disorders. In some forms, the comfort and effectiveness of these therapies may be improved.

Invasive ventilation (IV) provides ventilatory support to patients that are no longer able to effectively breathe themselves and may be provided using a tracheostomy tube. In some forms, the comfort and effectiveness of these therapies may be improved.

### 2.2.2.2 Flow therapies

Not all respiratory therapies aim to deliver a prescribed therapeutic pressure. Some respiratory therapies aim to deliver a prescribed respiratory volume, by delivering an inspiratory flow rate profile over a targeted duration, possibly superimposed on a positive baseline pressure. In other cases, the interface to the patient's airways is 'open' (unsealed) and the respiratory therapy may only supplement the patient's own spontaneous breathing with a flow of conditioned or enriched gas. In one example, High Flow therapy (HFT) is the provision of a continuous, heated, humidified flow of air to an entrance to the airway through an unsealed or open patient interface at a "treatment flow rate" that is held approximately constant throughout the respiratory cycle. The treatment flow rate is nominally set to exceed the patient's peak inspiratory flow rate. HFT has been used to treat OSA, CSR, respiratory failure, COPD, and other respiratory disorders. One mechanism of action is that the high flow rate of air at the airway entrance improves ventilation efficiency by flushing, or

washing out, expired $CO_2$ from the patient's anatomical deadspace. Hence, HFT is thus sometimes referred to as a deadspace therapy (DST). Other benefits may include the elevated warmth and humidification (possibly of benefit in secretion management) and the potential for modest elevation of airway pressures. As an alternative to constant flow rate, the treatment flow rate may follow a profile that varies over the respiratory cycle.

Another form of flow therapy is long-term oxygen therapy (LTOT) or supplemental oxygen therapy. Doctors may prescribe a continuous flow of oxygen enriched gas at a specified oxygen concentration (from 21%, the oxygen fraction in ambient air, to 100%) at a specified flow rate (e.g., 1 litre per minute (LPM), 2 LPM, 3 LPM, etc.) to be delivered to the patient's airway.

### 2.2.2.3 Supplementary oxygen

For certain patients, oxygen therapy may be combined with a respiratory pressure therapy or HFT by adding supplementary oxygen to the pressurised flow of air. When oxygen is added to respiratory pressure therapy, this is referred to as RPT with supplementary oxygen. When oxygen is added to HFT, the resulting therapy is referred to as HFT with supplementary oxygen.

### 2.2.3 Respiratory therapy Systems

These respiratory therapies may be provided by a respiratory therapy system or device. Such systems and devices may also be used to screen, diagnose, or monitor a condition without treating it.

A respiratory therapy system may comprise a Respiratory Pressure Therapy Device (RPT device), an air circuit, a humidifier, a patient interface, an oxygen source, and data management.

Another form of therapy system is a mandibular repositioning device.

### 2.2.3.1 Patient Interface

A patient interface may be used to interface respiratory equipment to its wearer, for example by providing a flow of air to an entrance to the airways. The flow of air may be provided via a mask to the nose and/or mouth, a tube to the mouth or a tracheostomy tube to the trachea of a patient. Depending upon the therapy to be applied, the patient interface may form a seal, e.g., with a region of the patient's face, to facilitate the delivery of gas at a pressure at sufficient variance with ambient pressure to effect therapy, e.g., at a positive pressure of about 10 $cmH_2O$ relative to ambient pressure. For other forms of therapy, such as the delivery of oxygen, the patient interface may not include a seal sufficient to facilitate delivery to the airways of a supply of gas at a positive pressure of about 10 $cmH_2O$. For flow therapies such as nasal HFT, the patient interface is configured to insufflate the nares but specifically to avoid a complete seal. One example of such a patient interface is a nasal cannula.

Certain other mask systems may be functionally unsuitable for the present field. For example, purely ornamental masks may be unable to maintain a suitable pressure. Mask systems used for underwater swimming or diving may be configured to guard against ingress of water from an external higher pressure, but not to maintain air internally at a higher pressure than ambient.

Certain masks may be clinically unfavourable for the present technology e.g. if they block airflow via the nose and only allow it via the mouth.

Certain masks may be uncomfortable or impractical for the present technology if they require a patient to insert a portion of a mask structure in their mouth to create and maintain a seal via their lips.

Certain masks may be impractical for use while sleeping, e.g. for sleeping while lying on one's side in bed with a head on a pillow.

While a mask for other applications (e.g. aviators) may not be suitable for use in treating sleep disordered breathing, a mask designed for use in treating sleep disordered breathing may be suitable for other applications.

For these reasons, patient interfaces for delivery of CPAP during sleep, NIV or IV form a distinct field.

### *2.2.3.1.1 Seal-forming structure*

Patient interfaces may include a seal-forming structure. Since it is in direct contact with the patient's face, the shape and configuration of the seal-forming structure can have a direct impact the effectiveness and comfort of the patient interface.

A patient interface may be partly characterised according to the design intent of where the seal-forming structure is to engage with the face in use. In one form of patient interface, a seal-forming structure may comprise a first sub-portion to form a seal around the left naris and a second sub-portion to form a seal around the right naris. In one form of patient interface, a seal-forming structure may comprise a single element that surrounds both nares in use. Such single element may be designed to for example overlay an upper lip region and a nasal bridge region of a face. In one form of patient interface a seal-forming structure may comprise an element that surrounds a mouth region in use, e.g. by forming a seal on a lower lip region of a face. In one form of patient interface, a seal-forming structure may comprise a single element that surrounds both nares and a mouth region in use. These different types of patient interfaces may be known by a variety of names by their manufacturer including nasal masks, full-face masks, nasal pillows, nasal puffs and oro-nasal masks.

### 2.2.3.1.2 Positioning and stabilising

A seal-forming structure of a patient interface used for positive air pressure therapy is subject to the corresponding force of the air pressure to disrupt a seal. Thus a variety of techniques have been used to position the seal-forming structure, and to maintain it in sealing relation with the appropriate portion of the face.

One technique is the use of adhesives. See for example US Patent Application Publication No. US 2010/0000534. However, the use of adhesives may be uncomfortable for some.

Another technique is the use of one or more straps and/or stabilising harnesses. Many such harnesses suffer from being one or more of ill-fitting, bulky, uncomfortable and awkward to use.

### 2.2.3.2 Respiratory Pressure Therapy (RPT) Device

A respiratory pressure therapy (RPT) device may be used individually or as part of a system to deliver one or more of a number of therapies described above, such as by operating the device to generate a flow of air for delivery to an interface to the airways. The flow of air may be pressure-controlled (for respiratory pressure therapies) or flow-controlled (for flow therapies such as HFT). Thus RPT devices may also act as flow therapy devices. Examples of RPT devices include a CPAP device and a ventilator.

Air pressure generators are known in a range of applications, e.g. industrial-scale ventilation systems. However, air pressure generators for medical applications have particular requirements not fulfilled by more generalised air pressure generators, such as the reliability, size and weight requirements of medical devices. In addition, even devices designed for medical treatment may suffer from shortcomings, pertaining to one or more of: comfort, noise, ease of use, efficacy, size, weight, manufacturability, cost, and reliability.

An example of the special requirements of certain RPT devices is acoustic noise.

Table of noise output levels of prior RPT devices (one specimen only, measured using test method specified in ISO 3744 in CPAP mode at 10 cmH$_2$O).

| RPT Device name | A-weighted sound pressure level dB(A) | Year (approx.) |
|---|---|---|
| C-Series Tango™ | 31.9 | 2007 |
| C-Series Tango™ with Humidifier | 33.1 | 2007 |
| S8 Escape™ II | 30.5 | 2005 |
| S8 Escape™ II with H4i™ Humidifier | 31.1 | 2005 |
| S9 AutoSet™ | 26.5 | 2010 |
| S9 AutoSet™ with H5i Humidifier | 28.6 | 2010 |

One known RPT device used for treating sleep disordered breathing is the S9 Sleep Therapy System, manufactured by ResMed Limited. Another example of an RPT device is a ventilator. Ventilators such as the ResMed Stellar™ Series of Adult and Paediatric Ventilators may provide support for invasive and non-invasive non-dependent ventilation for a range of patients for treating a number of conditions such as but not limited to NMD, OHS and COPD. Other examples of ventilators include the ResMed Lumis™ Series of non-invasive ventilators and the ResMed Astral™ series of life support ventilators.

The ResMed Elisée™ 150 ventilator and ResMed VS III™ ventilator may provide support for invasive and non-invasive dependent ventilation suitable for adult or paediatric patients for treating a number of conditions. These ventilators provide volumetric and barometric ventilation modes with a single or double limb circuit. RPT devices typically comprise a pressure generator, such as a motor-driven blower or a compressed gas reservoir, and are configured to supply a flow of air to the airway of a patient. In some cases, the flow of air may be supplied to the airway of the patient at positive pressure. The outlet of the RPT device is connected via an air circuit to a patient interface such as those described above.

The designer of a device may be presented with an infinite number of choices to make. Design criteria often conflict, meaning that certain design choices are far from routine or inevitable. Furthermore, the comfort and efficacy of certain aspects may be highly sensitive to small, subtle changes in one or more parameters.

### 2.2.3.3 Air circuit

An air circuit is a conduit or a tube constructed and arranged to allow, in use, a flow of air to travel between two components of a respiratory therapy system such as the RPT device and the patient interface. In some cases, there may be separate limbs of the air circuit for inhalation and exhalation. In other cases, a single limb air circuit is used for both inhalation and exhalation.

### 2.2.3.4 Humidifier

Delivery of a flow of air without humidification may cause drying of airways. The use of a humidifier with an RPT device and the patient interface produces humidified gas that minimizes drying of the nasal mucosa and increases patient airway comfort. In addition, in cooler climates, warm air applied generally to the face area in and about the patient interface is more comfortable than cold air. Humidifiers therefore often have the capacity to heat the flow of air was well as humidifying it.

A range of artificial humidification devices and systems are known; however, they may not fulfil the specialised requirements of a medical humidifier.

Medical humidifiers are used to increase humidity and/or temperature of the flow of air in relation to ambient air when required, typically where the patient may be asleep or resting (e.g. at a hospital). A medical humidifier for bedside placement may be small. A medical humidifier may be configured to only humidify and/or heat the flow of air delivered to the patient without humidifying and/or heating the patient's surroundings. Room-based systems (e.g. a sauna, an air conditioner, or an evaporative cooler), for example, may also humidify air that is breathed in by the patient, however those systems would also humidify and/or heat the entire room, which may cause discomfort to the occupants. Furthermore, medical humidifiers may have more stringent safety constraints than industrial humidifiers

While a number of medical humidifiers are known, they can suffer from one or more shortcomings. Some medical humidifiers may provide inadequate humidification, while some are difficult or inconvenient to use by patients.

### 2.2.3.5 Oxygen source

Experts in this field have recognized that exercise for respiratory failure patients provides long term benefits that slow the progression of the disease, improve quality of life and extend patient longevity. Most stationary forms of exercise like tread mills and stationary bicycles, however, are too strenuous for these patients. As a result, the need for mobility has long been recognized. Until recently, this mobility has been facilitated by the use of small compressed oxygen tanks or cylinders mounted on a cart with dolly wheels. The disadvantage of these tanks is that they contain a finite amount of oxygen and are heavy, weighing about 50 pounds when mounted.

Oxygen concentrators have been in use for about 50 years to supply oxygen for respiratory therapy. Traditional oxygen concentrators have been bulky and heavy making ordinary ambulatory activities with them difficult and impractical. Recently, companies that manufacture large stationary oxygen concentrators began developing portable oxygen concentrators (POCs). The advantage of POCs is that they can produce a theoretically endless supply of oxygen. In order to make these devices small for mobility, the various systems necessary for the production of oxygen enriched gas are condensed. POCs seek to utilize their produced oxygen as efficiently as possible, in order to minimise weight, size, and power consumption. This may be achieved by delivering the oxygen as series of pulses or "boli", each bolus timed to coincide with the start of inspiration. This therapy mode is known as pulsed or demand (oxygen) delivery (POD), in contrast with traditional continuous flow delivery more suited to stationary oxygen concentrators.

### 2.2.3.6 Data Management

There may be clinical reasons to obtain data to determine whether the patient prescribed with respiratory therapy has been "compliant", e.g. that the patient has used their RPT device according to one or more "compliance rules". One example of a compliance rule for CPAP therapy is that a patient, in order to be deemed compliant, is required to use the RPT device for at least four hours a night for at least 21 of 30 consecutive days. In order to determine a patient's compliance, a provider of the RPT device, such as a health care provider, may manually obtain data describing the patient's therapy using the RPT device, calculate the usage over a predetermined time period, and compare with the compliance rule. Once the health care provider has determined that the patient has used their RPT device according to the compliance rule, the health care provider may notify a third party that the patient is compliant.

There may be other aspects of a patient's therapy that would benefit from communication of therapy data to a third party or external system.

Existing processes to communicate and manage such data can be one or more of costly, time-consuming, and error-prone.

### 2.2.3.7 Vent technologies

Some forms of treatment systems may include a vent to allow the washout of exhaled carbon dioxide. The vent may allow a flow of gas from an interior space of a patient interface, e.g., the plenum chamber, to an exterior of the patient interface, e.g., to ambient.

The vent may comprise an orifice and gas may flow through the orifice in use of the mask. Many such vents are noisy. Others may become blocked in use and thus provide insufficient washout. Some vents may be disruptive of the sleep of a bed partner 1100 of the patient 1000, e.g. through noise or focussed airflow.

ResMed Limited has developed a number of improved mask vent technologies. See International Patent Application Publication No. WO 1998/034,665; International Patent Application Publication No. WO 2000/078,381; US Patent No. 6,581,594; US Patent Application Publication No. US 2009/0050156; US Patent Application Publication No. 2009/0044808. Furthermore, is referred to WO2015/089582 which discloses a respiratory pressure therapy device with various sensors for treatment of various disorders.

### 2.2.4 Screening, Diagnosis, and Monitoring Systems

Polysomnography (PSG) is a conventional system for diagnosis and monitoring of cardio-pulmonary disorders, and typically involves expert clinical staff to apply the system. PSG typically involves the placement of 15 to 20 contact sensors on a patient in order to record various bodily signals such as electroencephalography (EEG), electrocardiography (ECG), electrooculograpy (EOG), electromyography (EMG), etc. PSG for sleep disordered breathing has involved two nights of observation of a patient in a clinic, one night of pure diagnosis and a second night of titration of treatment parameters by a clinician. PSG is therefore expensive and inconvenient. In particular, it is unsuitable for home screening / diagnosis / monitoring of sleep disordered breathing.

Screening and diagnosis generally describe the identification of a condition from its signs and symptoms. Screening typically gives a true / false result indicating whether or not a patient's SDB is severe enough to warrant further investigation, while diagnosis may result in clinically actionable information. Screening and diagnosis tend to be one-off processes, whereas monitoring the progress of a condition can continue indefinitely. Some screening / diagnosis systems are suitable only for screening / diagnosis, whereas some may also be used for monitoring.

Clinical experts may be able to screen, diagnose, or monitor patients adequately based on visual observation of PSG signals. However, there are circumstances where a clinical expert may not be available, or a clinical expert may not be affordable. Different clinical experts may disagree on a patient's condition. In addition, a given clinical expert may apply a different standard at different times.

### 3 BRIEF SUMMARY OF THE TECHNOLOGY

The present technology is directed towards providing medical devices used in the screening, diagnosis, monitoring, amelioration, treatment, or prevention of respiratory disorders having one or more of improved comfort, cost, efficacy, ease of use and manufacturability.

A first aspect of the present technology relates to apparatus used in the screening, diagnosis, monitoring, amelioration, treatment or prevention of a respiratory disorder.

Another aspect of the present technology relates to methods used in the screening, diagnosis, monitoring, amelioration, treatment or prevention of a respiratory disorder.

An aspect of certain forms of the present technology is to provide methods and/or apparatus that improve the compliance of patients with respiratory therapy.

An aspect of one form of the present technology is to provide a method of detecting user interaction with a respiratory therapy device.

An aspect of one form of the present technology is a respiratory therapy device for treatment of sleep disordered breathing, the device comprising:

at least one sensor, configured to detect the presence of a user, wherein, on detection of the user, the device is configured to effect an action.

Another aspect of one form of the present technology is a device for supplying a flow of breathable gas at a positive pressure for respiratory therapy, the device comprising:

a pressure generator for generating the flow of breathable gas and supplying the flow to an outlet; and

a sensor configured to detect the presence of a user in proximity to the device,

wherein, on detection of the user by the sensor, the device is configured to effect an action.

In examples, the device may comprise a contact portion. In examples, the contact portion may be configured to be contacted directly or indirectly by the user to facilitate movement or use of the device.

In examples, the sensor may be configured to detect the presence of a user in proximity to the contact portion, such as contacting the contact portion or being in range of the sensor proximate to the contact portion. For example, the contact portion may be touched, gripped or held by the user while moving the device.

In examples, the contact portion may comprise one or more markings or structures defining or indicating to the user a region proximate the device within which the user's movements may be detected by the sensor.

In examples, the device may comprise a housing. The housing may contain or have mounted thereto one or more components of the device. For example, the housing may contain or have mounted thereto the sensor.

In examples, the housing may comprise an outer surface, and the contact portion may be provided on the outer surface of the housing.

In examples, the sensor may be a proximity sensor. For example, the sensor may comprise capacitive, inductive, optical, radar, sonar, ultrasonic, or magnetic sensing technologies as should be known to those skilled in the art. Use of a proximity sensor may advantageously allow for detection of a user as they approach, or a part of their body approaches, the contact portion(s) of the respiratory therapy device. For example, the sensor may be able to detect a user as they approach the device and effect an action accordingly.

In some examples, the proximity sensor may be configured to effect an action once motion of the patient's body, including specific gestures of part of the patient's body, or a sequence of motions or gestures, are detected. For example, the proximity sensor may be configured to detect a user moving towards the device, moving away from the device, or gesturing to the device such as waving, moving their hand laterally or longitudinally across the device, in circular motions, or any other suitable gestures such as pinching, pointing or rotating their hands or fingers.

In examples, the device may be configured to distinguish between one or more motions or gestures by the user. For example, the device may be configured to effect a first action in response to detection of a first motion or gesture by the sensor, and a second action in response to detection of a second motion or gesture by the sensor. In examples, the first action may be different to the second action. In other examples, the first action may be the same as the second action.

In examples, the sensor may be configured to detect user movement in front of the device. Additionally, or alternatively, the sensor may be configured to detect user movement above the device or proximate to one or more sides of the device. In other examples the sensor may be configured to detect user movement in any direction relative to the device.

Potential advantages of using a proximity sensor may include:

- The ability to position the proximity sensor within the housing of the device. This may advantageously allow the sensor to be protected by the housing of the device;

- Greater positioning flexibility for the sensor, for example the sensor may be positioned behind, adjacent to or opposite the contact portion;

- Greater detection coverage, i.e. detection coverage which is not limited to the surface of the device, for example the ability to detect a user's or the user's hand or fingers as they approach the device, gesture to the device or enter a cavity of the device;

- Providing the device with a sleek appearance; and/or

- Simplified device construction, for example the sensor may be positioned on an internal circuit board of the device, such as a circuit board used for one or more additional device functions.

In other examples, the sensor may be a contact sensor. For example, the sensor may comprise a mechanical activation mechanism (such as a button), electrical contacts, capacitive, inductive, piezo-electric resistive sensors, including touch-screen technologies. The use of a contact sensor may advantageously provide a lower cost sensing solution compared to a proximity sensor, for example. Additional potential advantages include reduced potential for false detections.

In examples, where the sensor is a contact sensor, one or more components of the sensor may be positioned externally to the housing. For example, the contact sensor may comprise one or more contact surfaces on an external surface of the housing. In examples, the contact surfaces may extend through one or more apertures of the housing. For example, the contact surfaces may be electrically connected to one or more components of the sensor which are housed within the housing of the device. In other examples, the contact sensor may be positioned entirely outside of the housing.

For example, the contact sensor may be integrated with an external label on the device.

In examples, the contact portion of the device may comprise one or more regions of the device. For example, the contact portion of the device may comprise a region of the device which facilitates movement of the device, such as lifting. For example, the contact portion may comprise a gripping portion or cavity configured to receive a hand, fingers, thumb or limb of the user. In other examples, the contact portion may include one or more surfaces of the device. For example, the contact portion may be a top surface of the device, and / or one or more side, front or back surfaces of the device. In examples, the contact portion may include a protrusion, rib or textured surface in order to assist the user in moving the device. In examples, the contact portion may comprise a handle.

In examples, the device may comprise one or more input device(s). For example, the input device(s) may comprise one or more of buttons, dials, switches, touch-screen interfaces, microphones, or video input devices.

In examples, the device may comprise one or more output device(s) such as a display, light emitting diode (LED), buzzer, speaker or vibrator.

In examples, the device may comprise combinations of input device(s) and output device(s) in order to provide a user interface. For example, the user interface may enable a user to configure and operate the device.

In examples, the respiratory therapy device may include an integral humidifier. In other examples, the humidifier may be a separate unit to the respiratory therapy device but fluidly connected thereto, for example by means of a fluid conduit. In other examples, the humidifier and RPT device may be able to be assembled and disassembled where, in the assembled configuration they form a single device. In other words, the humidifier may be releasably connected to the device.

In examples, the device may be configured to communicate with one or more remote, or secondary devices or systems. For example, using a wireless network such as WiFi or Bluetooth.

In examples, the sensor is configured to detect a user interacting with the contact portion of the device. For example, the sensor may detect when a user is near to the contact portion, has touched the contact portion or has interacted with a handle or recess of the device. In examples, the device may effect one or more actions in response to detection of the user.

In examples, the device may comprise one or more releasable components. For example, the releasable component may be a water reservoir, humidifier or conduit.

In examples, the device may effect a change within the device itself. In other examples, the device may effect a change in one or more releasable component of the device (such as a humidifier) or secondary devices or systems (such as standalone devices connected to the device). For example, the device may send a signal to a secondary device in order to effect a change in the secondary device. In examples, the device may communicate with a secondary device positioned upstream or downstream of the device. For example, the device may communicate with a connected humidifier, blower, conduit or patient interface.

In examples, the signal may be a wired signal, such as an active-high or active-low electrical signal. In other examples, the signal may be transmitted using one or more communication protocol such as $I^2C$, serial, or Ethernet. In other examples, the signal may be a wireless signal such as WiFi, Bluetooth, Zigbee etc. For example, the device may wirelessly communicate with a remote secondary device such as a tablet, PDA or phone to effect a change.

In examples, the action effected may comprise disabling one or more features of the device. In examples, the action effected may include one or more of:

- Disabling one or more features of an input device. For example, the buttons, and/or touch-screen interface may be disabled to prevent inadvertent button presses as the device is being moved or transported, i.e. the touch functionality of a touch-screen interface may be disabled;

- Disabling one or more features of an output device. For example, a display or a backlight of the display may be turned off to save power. Alternatively, or additionally, the device may be configured to silence an alarm condition when a user is detected interacting with a contact portion i.e. an indicator light and/or any audible alarms may be silenced/disabled;

- Providing one or more messages, alerts or other feedback to the user indicating to the user that the device should not be moved while in use. For example, the device may alert the user via one or more output device(s) of the device such as the display, an indicator light or a speaker. In other examples, the device may alert the user via one or more secondary, for example peripheral or remote devices. For example, the device may be configured to cause an audible, visual or tactile alert to be played via the user's cell phone. Such an action may be effected by the sensor sending a signal to a controller to indicate that the user has been detected, and the controller being configured to cause a transmitting device to send an actuation signal to a remote device such as a cell phone. In some forms the messages, alerts or other feedback may be provided through a combination of devices. In some forms the feedback may take the form of a message having text informing the user that the device should not be moved while in use. In other forms the feedback may take the form of an alert that implies this to the user, for example by illuminating a light associated with a warning message or symbol understood to be indicating not to move the device in use.

- Providing one or more messages, alerts or other feedback to the user concerning one or more releasable components connected to the device. For example, the device may notify or alert the user to the presence of absence of the releasable component, such as notifying the user that a conduit, water reservoir or humidifier is connected to the inlet and/or outlet. For example, the device may notify the user via one or more of the output devices of the device and/or a secondary device such as a phone or tablet. The user may be notified through any one or more of visual, audible or tactile notifications as indicated in the previous example;

- Providing one or more messages, alerts or other feedback to the user concerning any aspect of the use and/or operation of the RPT device, a humidifier, and/or a peripheral device; and

- Changing one or more operating parameters of the device or secondary device. For example, the device may determine that the user of the device is awake and lower the therapy pressure delivered by the device to improve user comfort.

In examples, the contact portion may comprise a protrusion. For example, the protrusion may extend outwardly from a top surface of the housing. The protrusion may further provide a cavity or overhang. The protrusion or overhang may be configured to receive a user's hand in use. For example, the protrusion may extend outwardly from the top surface of the housing at an acute angle. In examples the acute angle may be between approximately 10 degrees and approximately 40 degrees. In other examples, the angle may be between approximately 20 degrees and 30 degrees.

In examples, the protrusion may comprise a gripping portion. For example, the gripping portion may be a rib. The rib may be provided to an underside surface of the protrusion to aid a user in lifting or moving the device. For example, the rib may be provided on a distal part of the protrusion, away from the main body of the device.

In examples, the rib may comprise the sensor i.e. the sensor may be positioned within or on a surface of the rib. In other examples, the sensor may be positioned underneath the protrusion within or on the body of the device i.e. on or inside housing.

In examples, the protrusion may comprise at least one input or output device such as a button, display or touch-screen interface. Positioning at least one input or output device on the protrusion may advantageously assist in positioning the input or output device at an angle which facilitates use. For example, the protrusion may extend from the housing at an angle of between approximately 20 degrees and approximately 45 degrees. In other examples the angle may be between approximately 25 degrees and approximately 35 degrees.

An aspect of one form of the present technology is a method of manufacturing apparatus.

An aspect of certain forms of the present technology is a medical device that is easy to use, e.g. by a person who does not have medical training, by a person who has limited dexterity, vision or by a person with limited experience in using this type of medical device.

An aspect of one form of the present technology is a portable RPT device that may be carried by a person, e.g., around the home of the person.

An aspect of one form of the present technology is a patient interface that may be washed in a home of a patient, e.g., in soapy water, without requiring specialised cleaning equipment. An aspect of one form of the present technology is a humidifier tank that may be washed in a home of a patient, e.g., in soapy water, without requiring specialised cleaning equipment.

The methods, systems, devices and apparatus described may be implemented so as to improve the functionality of a processor, such as a processor of a specific purpose computer, respiratory monitor and/or a respiratory therapy apparatus. Moreover, the described methods, systems, devices and apparatus can provide improvements in the technological field of automated management, monitoring and/or treatment of respiratory conditions, including, for example, sleep disordered breathing.

Of course, portions of the aspects may form sub-aspects of the present technology. Also, various ones of the sub-aspects and/or aspects may be combined in various manners and also constitute additional aspects or sub-aspects of the present technology.

Other features of the technology will be apparent from consideration of the information contained in the following detailed description, abstract, drawings and claims.

4 BRIEF DESCRIPTION OF THE DRAWINGS

The present technology is illustrated by way of example, and not by way of limitation, in the figures of the accompanying drawings, in which like reference numerals refer to similar elements including:

4.1 RESPIRATORY THERAPY SYSTEMS

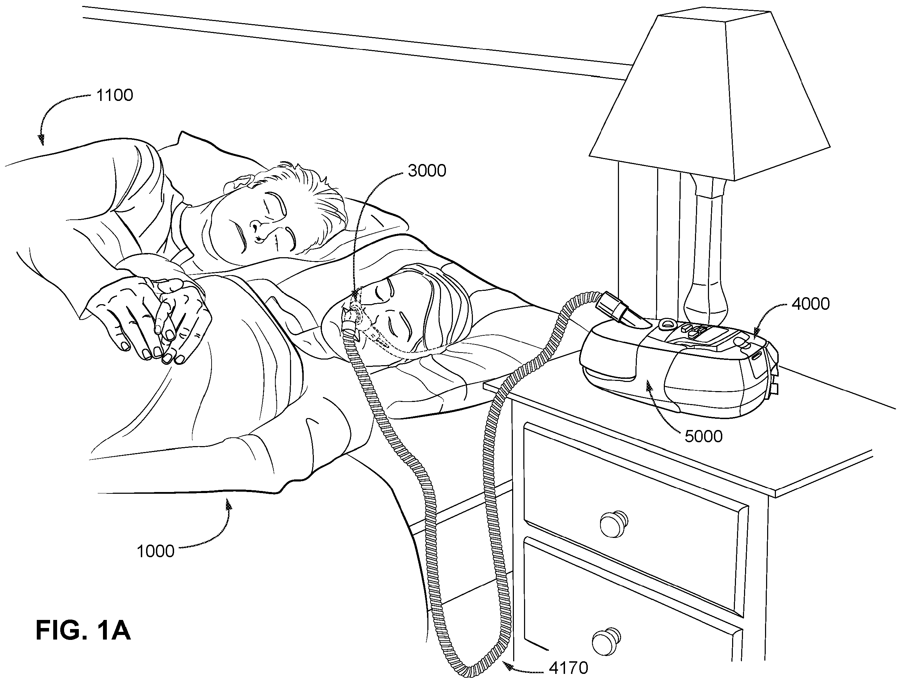

Fig. 1A shows a system including a patient 1000 wearing a patient interface 3000, in the form of nasal pillows, receiving a supply of air at positive pressure from an RPT device 4000. Air from the RPT device 4000 is conditioned in a humidifier 5000, and passes along an air circuit 4170 to the patient 1000. A bed partner 1100 is also shown. The patient is sleeping in a supine sleeping position.

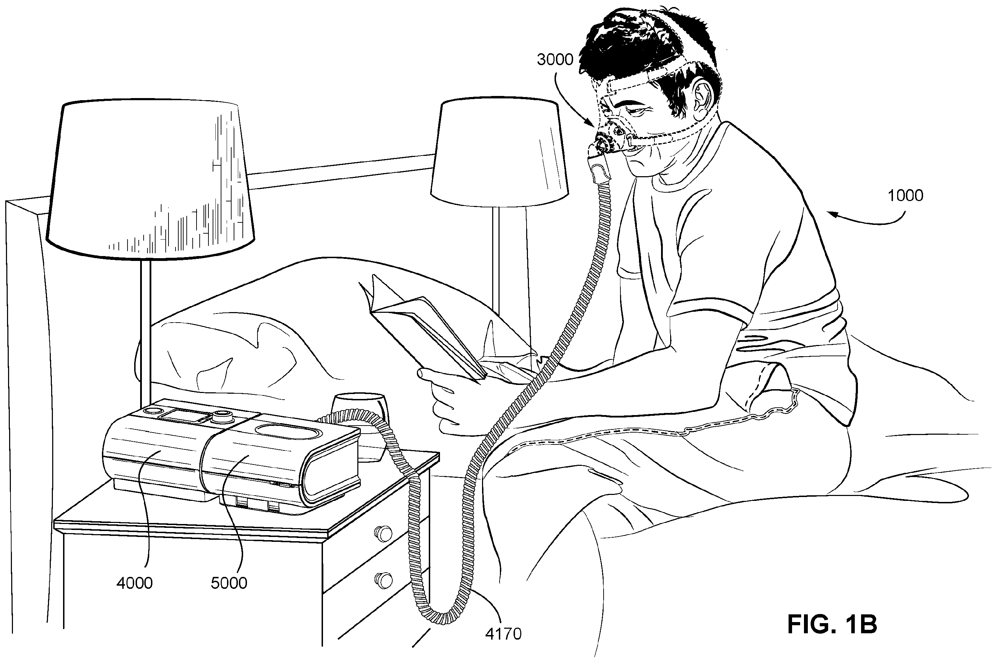

Fig. 1B shows a system including a patient 1000 wearing a patient interface 3000, in the form of a nasal mask, receiving a supply of air at positive pressure from an RPT device 4000. Air from the RPT device is humidified in a humidifier 5000, and passes along an air circuit 4170 to the patient 1000.

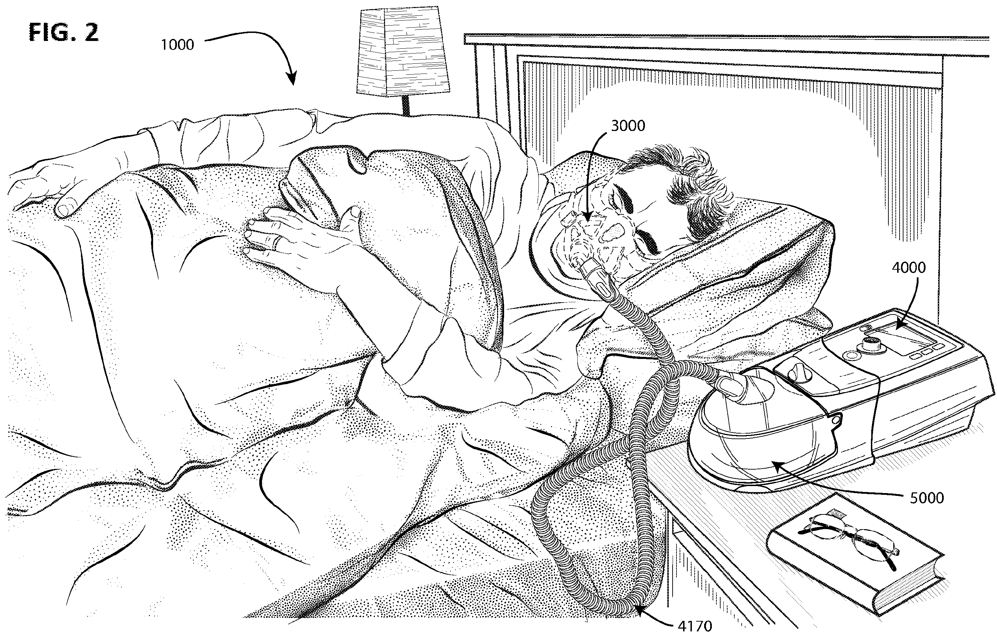

Fig. 2 shows a system including a patient 1000 wearing a patient interface 3000, in the form of a full-face mask, receiving a supply of air at positive pressure from an RPT device 4000. Air from the RPT device is humidified in a humidifier 5000, and passes along an air circuit 4170 to the patient 1000. The patient is sleeping in a side sleeping position.

4.2 PATIENT INTERFACE

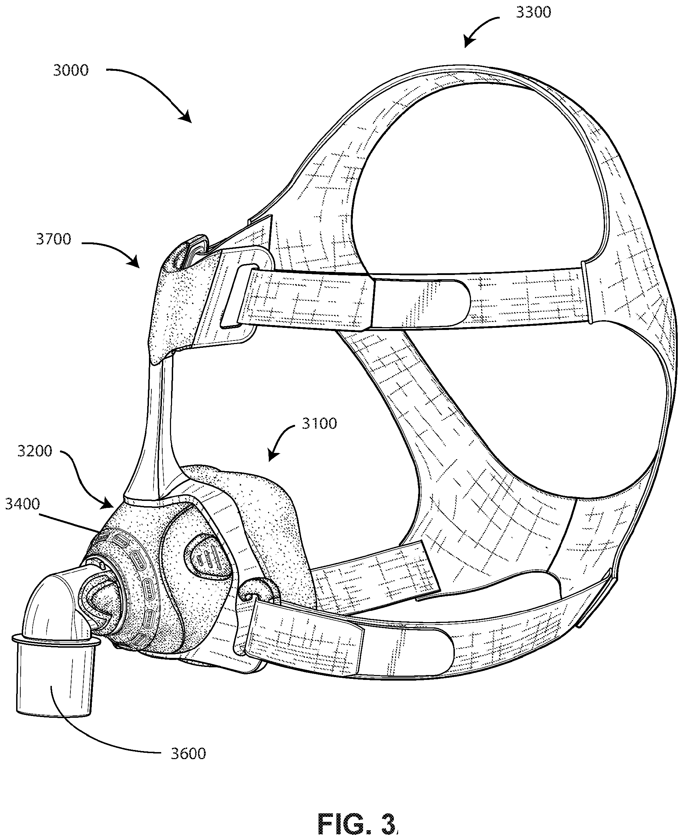

Fig. 3 shows a patient interface in the form of a nasal mask in accordance with one form of the present technology.

4.3 RPT DEVICE

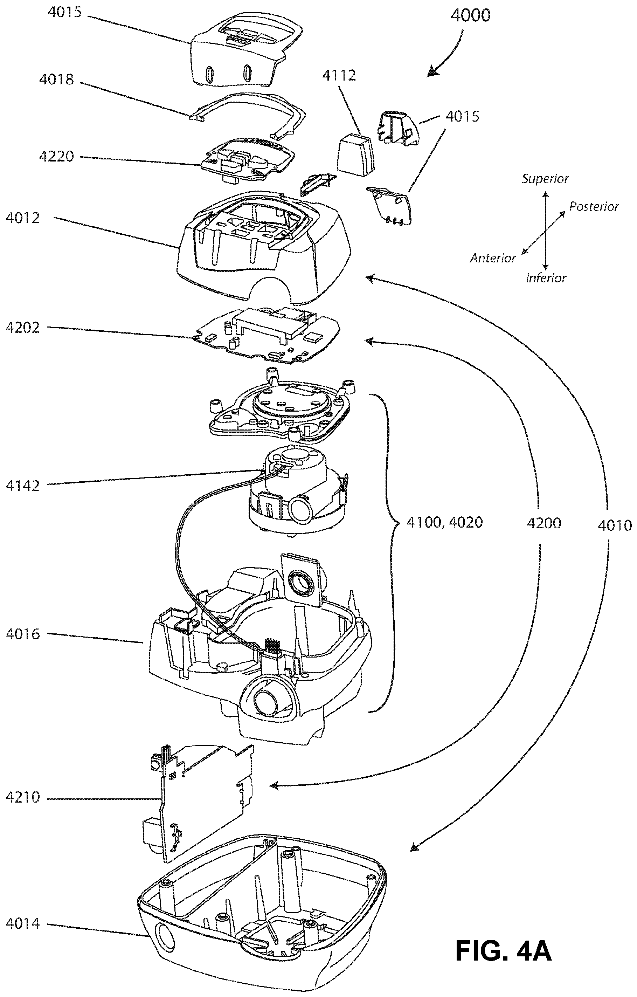

Fig. 4A shows an RPT device in accordance with one form of the present technology.

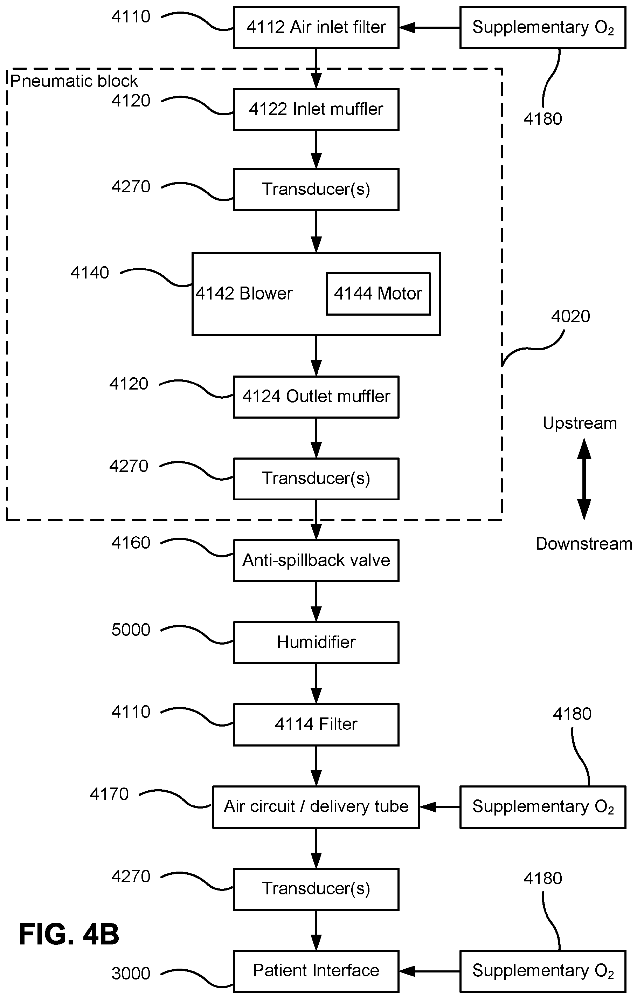

Fig. 4B is a schematic diagram of the pneumatic path of an RPT device in accordance with one form of the present technology. The directions of upstream and downstream are indicated with reference to the blower and the patient interface. The blower is defined to be upstream of the patient interface and the patient interface is defined to be downstream of the blower, regardless of the actual flow direction at any particular moment. Items which are located within the pneumatic path between the blower and the patient interface are downstream of the blower and upstream of the patient interface.

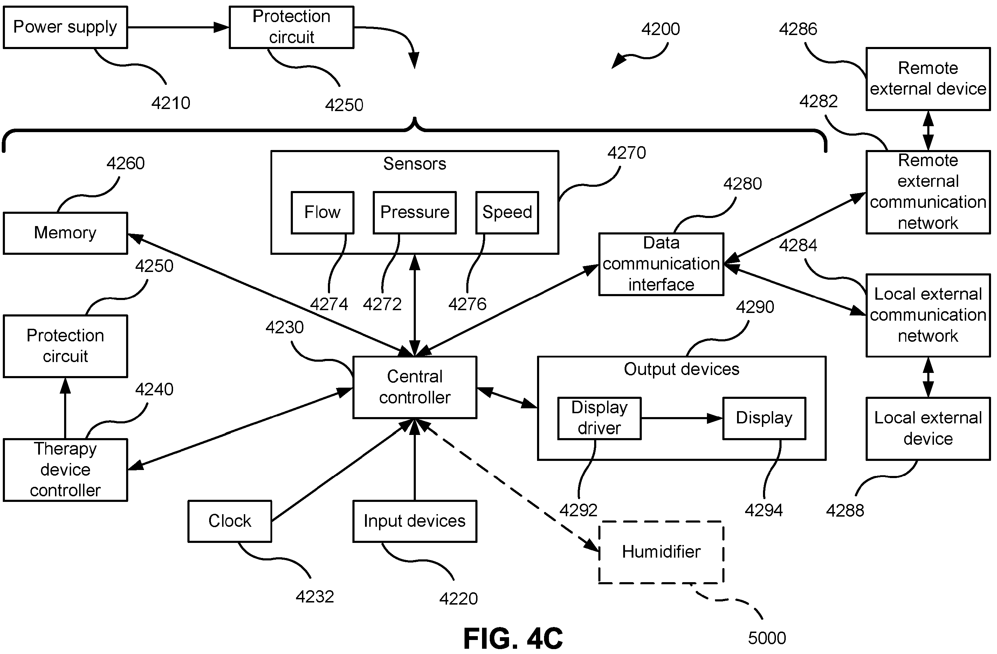

Fig. 4C is a schematic diagram of the electrical components of an RPT device in accordance with one form of the present technology.

Fig. 5A shows an isometric view of a humidifier in accordance with one form of the present technology.

Fig. 5B shows an isometric view of a humidifier in accordance with one form of the present technology, showing a humidifier reservoir 5110 removed from the humidifier reservoir dock 5130.

Fig. 5C shows a schematic of a humidifier in accordance with one form of the present technology.

Fig. 6 shows an isometric view of a respiratory therapy device in accordance with one form of the present technology.

Fig. 7 shows a block diagram of a control system according to one form of the present technology.

Fig. 8A shows an isometric view of a respiratory therapy device comprising a water reservoir in accordance with one form of the present technology.

Fig. 8B shows the respiratory therapy device of Fig. 8A with the water reservoir removed.

Fig. 8C shows a further isometric view of the respiratory therapy device of Figs. 8A and 8B.

Fig. 8D shows a further isometric view of the respiratory therapy device of Figs. 8A, 8B and 8C.

Fig. 9A shows a respiratory therapy device in accordance with one form of the present technology.

Fig. 9B shows the respiratory therapy device of Fig. 9A connected to a releasable component in the form of a humidifier.

Fig. 9C shows the respiratory therapy device and humidifier of Fig. 9B with the lid of the humidifier in an open position.

Fig. 10 shows a partial cross-sectional view of a contact portion according to the present technology.

## 5 DETAILED DESCRIPTION OF EXAMPLES OF THE TECHNOLOGY

Before the present technology is described in further detail, it is to be understood that the technology is not limited to the particular examples described herein, which may vary. It is also to be understood that the terminology used in this disclosure is for the purpose of describing only the particular examples discussed herein, and is not intended to be limiting.

The following description is provided in relation to various examples which may share one or more common characteristics and/or features. It is to be understood that one or more features of any one example may be combinable with one or more features of another example or other examples. In addition, any single feature or combination of features in any of the examples may constitute a further example.

### 5.1 THERAPY

In one form, the present technology comprises a method for treating a respiratory disorder comprising applying positive pressure to the entrance of the airways of a patient 1000.

In certain examples of the present technology, a supply of air at positive pressure is provided to the nasal passages of the patient via one or both nares.

In certain examples of the present technology, mouth breathing is limited, restricted or prevented.

### 5.2 RESPIRATORY THERAPY SYSTEMS

In one form, the present technology comprises a respiratory therapy system for treating a respiratory disorder. The respiratory therapy system may comprise an RPT device 4000 for supplying a flow of air to the patient 1000 via an air circuit 4170 and a patient interface 3000 or 3800.

### 5.3 PATIENT INTERFACE

A non-invasive patient interface 3000 in accordance with one aspect of the present technology comprises the following functional aspects: a seal-forming structure 3100, a plenum chamber 3200, a positioning and stabilising structure 3300, a vent 3400, one form of connection port 3600 for connection to air circuit 4170, and a forehead support 3700. In some forms a functional aspect may be provided by one or more physical components. In some forms, one physical component may provide one or more functional aspects. In use the seal-forming structure 3100 is arranged to surround an entrance to the airways of the patient so as to maintain positive pressure at the entrance(s) to the airways of the patient 1000. The sealed patient interface 3000 is therefore suitable for delivery of positive pressure therapy.

An unsealed patient interface 3800, in the form of a nasal cannula, includes nasal prongs 3810a, 3810b which can deliver air to respective nares of the patient 1000 via respective orifices in their tips. Such nasal prongs do not generally form a seal with the inner or outer skin surface of the nares. The air to the nasal prongs may be delivered by one or more air supply lumens 3820a, 3820b that are coupled with the nasal cannula 3800. The lumens 3820a, 3820b lead from the nasal cannula 3800 to a respiratory therapy device via an air circuit. The unsealed patient interface 3800 is particularly suitable for delivery of flow therapies, in which the RPT device generates the flow of air at controlled flow rates rather than controlled pressures. The "vent" at the unsealed patient interface 3800, through which excess airflow escapes to ambient, is the passage between the end of the prongs 3810a and 3810b of the cannula 3800 via the patient's nares to atmosphere.

If a patient interface is unable to comfortably deliver a minimum level of positive pressure to the airways, the patient interface may be unsuitable for respiratory pressure therapy.

The patient interface 3000 in accordance with one form of the present technology is constructed and arranged to be able to provide a supply of air at a positive pressure of at least 6 cmH$_2$O with respect to ambient.

The patient interface 3000 in accordance with one form of the present technology is constructed and arranged to be able to provide a supply of air at a positive pressure of at least 10 cmH$_2$O with respect to ambient.

The patient interface 3000 in accordance with one form of the present technology is constructed and arranged to be able to provide a supply of air at a positive pressure of at least 20 cmH$_2$O with respect to ambient.

5.4 RPT DEVICE

An RPT device 4000 in accordance with one aspect of the present technology comprises mechanical, pneumatic, and/or electrical components and is configured to execute one or more algorithms 4300, such as any of the methods, in whole or in part, described herein. The RPT device 4000 may be configured to generate a flow of air for delivery to a patient's airways, such as to treat one or more of the respiratory conditions described elsewhere in the present document.

In one form, the RPT device 4000 is constructed and arranged to be capable of delivering a flow of air in a range of -20 L/min to +150 L/min while maintaining a positive pressure of at least 6 cmH$_2$O, or at least 10cmH$_2$O, or at least 20 cmH$_2$O.

The RPT device may have an external housing 4010, formed in two parts, an upper portion 4012 and a lower portion 4014. Furthermore, the external housing 4010 may include one or more panel(s) 4015. The RPT device 4000 comprises a chassis 4016 that supports one or more internal components of the RPT device 4000. The RPT device 4000 may include a handle 4018.

The pneumatic path of the RPT device 4000 may comprise one or more air path items, e.g., an inlet air filter 4112, an inlet muffler 4122, a pressure generator 4140 capable of supplying air at positive pressure (e.g., a blower 4142), an outlet muffler 4124 and one or more transducers 4270, such as pressure sensors 4272 and flow rate sensors 4274.

One or more of the air path items may be located within a removable unitary structure which will be referred to as a pneumatic block 4020. The pneumatic block 4020 may be located within the external housing 4010. In one form a pneumatic block 4020 is supported by, or formed as part of, the chassis 4016.

The RPT device 4000 may have an electrical power supply 4210, one or more input devices 4220, a central controller 4230, a therapy device controller 4240, a pressure generator 4140, one or more protection circuits 4250, memory 4260, transducers 4270, data communication interface 4280 and one or more output devices 4290. Electrical components 4200 may be mounted on a single Printed Circuit Board Assembly (PCBA) 4202. In an alternative form, the RPT device 4000 may include more than one PCBA 4202.

### 5.4.1 RPT device mechanical & pneumatic components

An RPT device may comprise one or more of the following components in an integral unit. In an alternative form, one or more of the following components may be located as respective separate units.

### 5.4.1.1 Air filter(s)

An RPT device in accordance with one form of the present technology may include an air filter 4110, or a plurality of air filters 4110.

In one form, an inlet air filter 4112 is located at the beginning of the pneumatic path upstream of a pressure generator 4140.

In one form, an outlet air filter 4114, for example an antibacterial filter, is located between an outlet of the pneumatic block 4020 and a patient interface 3000 or 3800.

### 5.4.1.2 Muffler(s)

An RPT device in accordance with one form of the present technology may include a muffler 4120, or a plurality of mufflers 4120.

In one form of the present technology, an inlet muffler 4122 is located in the pneumatic path upstream of a pressure generator 4140.

In one form of the present technology, an outlet muffler 4124 is located in the pneumatic path between the pressure generator 4140 and a patient interface 3000 or 3800.

### 5.4.1.3 Pressure generator

In one form of the present technology, a pressure generator 4140 for producing a flow, or a supply, of air at positive pressure is a controllable blower 4142. For example, the blower 4142 may include a brushless DC motor 4144 with one or more impellers. The impellers may be located in a volute. The blower may be capable of delivering a supply of air, for example at a rate of up to about 120 litres/minute, at a positive pressure in a range from about 4 cmH$_2$O to about 20 cmH$_2$O, or in other forms up to about 30 cmH$_2$O when delivering respiratory pressure therapy. The blower may be as described in any one of the following patents or patent applications U.S. Patent No. 7,866,944; U.S. Patent No. 8,638,014; U.S. Patent No. 8,636,479; and PCT Patent Application Publication No. WO 2013/020167.

The pressure generator 4140 is under the control of the therapy device controller 4240.

In other forms, a pressure generator 4140 may be a piston-driven pump, a pressure regulator connected to a high pressure source (e.g. compressed air reservoir), or a bellows.

### 5.4.1.4 Transducer(s)

Transducers may be internal of the RPT device, or external of the RPT device. External transducers may be located for example on or form part of the air circuit, e.g., the patient interface. External transducers may be in the form of noncontact sensors or proximity sensors such as Doppler radar movement sensors that transmit or transfer data to the RPT device.

In one form of the present technology, one or more transducers 4270 are located upstream and/or downstream of the pressure generator 4140. The one or more transducers 4270 may be constructed and arranged to generate signals representing properties of the flow of air such as a flow rate, a pressure or a temperature at that point in the pneumatic path.

In one form of the present technology, one or more transducers 4270 may be located proximate to the patient interface 3000 or 3800.

In one form, a signal from a transducer 4270 may be filtered, such as by low-pass, high-pass or band-pass filtering.

#### 5.4.1.4.1 Flow rate sensor

A flow rate sensor 4274 in accordance with the present technology may be based on a differential pressure transducer, for example, an SDP600 Series differential pressure transducer from SENSIRION.

In one form, a signal generated by the flow rate sensor 4274 and representing a flow rate is received by the central controller 4230.

#### 5.4.1.4.2 Pressure sensor

A pressure sensor 4272 in accordance with the present technology is located in fluid communication with the pneumatic path. An example of a suitable pressure sensor is a transducer from the HONEYWELL ASDX series. An alternative suitable pressure sensor is a transducer from the NPA Series from GENERAL ELECTRIC.

In one form, a signal generated by the pressure sensor 4272 is received by the central controller 4230.

#### 5.4.1.4.3 Motor speed transducer

In one form of the present technology a motor speed transducer 4276 is used to determine a rotational velocity of the motor 4144 and/or the blower 4142. A motor speed signal from the motor speed transducer 4276 may be provided to the therapy device controller 4240. The motor speed transducer 4276 may, for example, be a speed sensor, such as a Hall effect sensor.

### 5.4.1.5 Anti-spill back valve

In one form of the present technology, an anti-spill back valve 4160 is located between the humidifier 5000 and the pneumatic block 4020. The anti-spill back valve is constructed and arranged to reduce the risk that water will flow upstream from the humidifier 5000, for example to the motor 4144.

### 5.4.2 RPT device electrical components

### 5.4.2.1 Power supply

A power supply 4210 may be located internal or external of the external housing 4010 of the RPT device 4000.

In one form of the present technology, power supply 4210 provides electrical power to the RPT device 4000 only. In another form of the present technology, power supply 4210 provides electrical power to both RPT device 4000 and humidifier 5000.

### 5.4.2.2 Input devices

In one form of the present technology, an RPT device 4000 includes one or more input devices 4220 in the form of buttons, switches or dials to allow a person to interact with the device. The buttons, switches or dials may be physical devices, or software devices accessible via a touch screen. The buttons, switches or dials may, in one form, be physically connected to the external housing 4010, or may, in another form, be in wireless communication with a receiver that is in electrical connection to the central controller 4230.

In one form, the input device 4220 may be constructed and arranged to allow a person to select a value and/or a menu option.

In one form of the present technology a sensor may be used to detect the presence of a user at or near to the device, and on detection of the user, effect an action, for example provide a signal which disables one or more features of the input device, as will be explained in more detail below.

### 5.4.2.3 Central controller

In one form of the present technology, the central controller 4230 is one or a plurality of processors suitable to control an RPT device 4000.

Suitable processors may include an x86 INTEL processor, a processor based on ARM® Cortex®-M processor from ARM Holdings such as an STM32 series microcontroller from ST MICROELECTRONIC. In certain alternative forms of the present technology, a 32-bit RISC CPU, such as an STR9 series microcontroller from ST MICROELECTRONICS or a 16-bit RISC CPU such as a processor from the MSP430 family of microcontrollers, manufactured by TEXAS INSTRUMENTS may also be suitable.

In one form of the present technology, the central controller 4230 is a dedicated electronic circuit.

In one form, the central controller 4230 is an application-specific integrated circuit. In another form, the central controller 4230 comprises discrete electronic components.

The central controller 4230 may be configured to receive input signal(s) from one or more transducers 4270, one or more input devices 4220, and the humidifier 5000.

The central controller 4230 may be configured to provide output signal(s) to one or more of an output device 4290, a therapy device controller 4240, a data communication interface 4280, and the humidifier 5000.

In some forms of the present technology, the central controller 4230 is configured to implement the one or more methodologies described herein, such as the one or more algorithms 4300 expressed as computer programs stored in a non-transitory computer readable storage medium, such as memory 4260. In some forms of the present technology, the central controller 4230 may be integrated with an RPT device 4000. However, in some forms of the present technology, some methodologies may be performed by a remotely located device. For example, the remotely located device may determine control settings for a ventilator or detect respiratory related events by analysis of stored data such as from any of the sensors described herein.

### 5.4.2.4 Clock

The RPT device 4000 may include a clock 4232 that is connected to the central controller 4230.

### 5.4.2.5 Therapy device controller

In one form of the present technology, therapy device controller 4240 is a therapy control module 4330 that forms part of the algorithms 4300 executed by the central controller 4230.

In one form of the present technology, therapy device controller 4240 is a dedicated motor control integrated circuit. For example, in one form a MC33035 brushless DC motor controller, manufactured by ONSEMI is used.

### 5.4.2.6 Protection circuits

The one or more protection circuits 4250 in accordance with the present technology may comprise an electrical protection circuit, a temperature and/or pressure safety circuit.

### 5.4.2.7 Memory

In accordance with one form of the present technology the RPT device 4000 includes memory 4260, e.g., non-volatile memory. In some forms, memory 4260 may include battery powered static RAM. In some forms, memory 4260 may include volatile RAM.

Memory 4260 may be located on the PCBA 4202. Memory 4260 may be in the form of EEPROM, or NAND flash.

Additionally, or alternatively, RPT device 4000 includes a removable form of memory 4260, for example a memory card made in accordance with the Secure Digital (SD) standard.

In one form of the present technology, the memory 4260 acts as a non-transitory computer readable storage medium on which is stored computer program instructions expressing the one or more methodologies described herein, such as the one or more algorithms 4300.

### 5.4.2.8 Data communication systems

In one form of the present technology, a data communication interface 4280 is provided, and is connected to the central controller 4230. Data communication interface 4280 may be connectable to a remote external communication network 4282 and/or a local external communication network 4284. The remote external communication network 4282 may be connectable to a remote external device 4286. The local external communication network 4284 may be connectable to a local external device 4288.

In one form, data communication interface 4280 is part of the central controller 4230. In another form, data communication interface 4280 is separate from the central controller 4230, and may comprise an integrated circuit or a processor.

In one form, remote external communication network 4282 is the Internet. The data communication interface 4280 may use wired communication (e.g. via Ethernet, or optical fibre) or a wireless protocol (e.g. CDMA, GSM, LTE) to connect to the Internet.

In one form, local external communication network 4284 utilises one or more communication standards, such as Bluetooth, or a consumer infrared protocol.

In one form, remote external device 4286 is one or more computers, for example a cluster of networked computers. In one form, remote external device 4286 may be virtual computers, rather than physical computers. In either case, such a remote external device 4286 may be accessible to an appropriately authorised person such as a clinician.

The local external device 4288 may be a personal computer, mobile phone, tablet or remote control.

### 5.4.2.9 Output devices including optional display, alarms

An output device 4290 in accordance with the present technology may take the form of one or more of a visual, audio and haptic unit. A visual display may be a Liquid Crystal Display (LCD) or Light Emitting Diode (LED) display.

#### 5.4.2.9.1 Display driver

A display driver 4292 receives as an input the characters, symbols, or images intended for display on the display 4294, and converts them to commands that cause the display 4294 to display those characters, symbols, or images.

#### 5.4.2.9.2 Display

A display 4294 is configured to visually display characters, symbols, or images in response to commands received from the display driver 4292. For example, the display 4294 may be an eight-segment display, in which case the display driver 4292 converts each character or symbol, such as the figure "0", to eight logical signals indicating whether the eight respective segments are to be activated to display a particular character or symbol.

In one form of the present technology a sensor may be used to detect the presence of a user at or near to the device, and on detection of the user, effect an action, for example provide a signal which disables one or more features of the output device, as will be explained in more detail below.

### 5.4.3 RPT device algorithms

As mentioned above, in some forms of the present technology, the central controller 4230 may be configured to implement one or more algorithms 4300 expressed as computer programs stored in a non-transitory computer readable storage medium, such as memory 4260. The algorithms 4300 are generally grouped into groups referred to as modules.

In other forms of the present technology, some portion or all of the algorithms 4300 may be implemented by a controller of an external device such as the local external device 4288 or the remote external device 4286. In such forms, data representing the input signals and / or intermediate algorithm outputs necessary for the portion of the algorithms 4300 to be executed at the external device may be communicated to the external device via the local external communication network 4284 or the remote external communication network 4282. In such forms, the portion of the algorithms 4300 to be executed at the external device may be expressed as computer programs stored in a non-transitory computer readable storage medium accessible to the controller of the external device. Such programs configure the controller of the external device to execute the portion of the algorithms 4300.

In such forms, the therapy parameters generated by the external device via the therapy engine module 4320 (if such forms part of the portion of the algorithms 4300 executed by the external device) may be communicated to the central controller 4230 to be passed to the therapy control module 4330.

### 5.4.3.1 Adaptive Learning Algorithms

One aspect of the present technology relates to the devices and methods of detecting a user in proximity to a respiratory therapy device. Certain forms of this technology rely on user detection via one or more proximity sensors. In these forms it may be advantageous to utilise an adaptive learning algorithm such as machine learning to aid in distinguishing between a user and nearby objects that are not a user, such as walls.

For example, adaptive learning may be used to reduce the rate of false detections by adjusting the detection algorithm based on one or more of: temperature; humidity; ambient light; time of day; sound; vibration; size, shape or speed of the detected object.

One or more adaptive learning algorithms may be used to generate parameters for detection of objects. These parameters may be programmed into the respiratory therapy device. Alternatively, or additionally, the device may include one or more adaptive algorithms which enable the detection algorithms to adjust in use.

In forms of the technology it may be desirable to use one or more adaptive learning algorithms to detect and determine whether a user's movement, or a user's interaction with an input device located in close proximity to a contact portion, was an action unrelated to the device, an action intended to trigger a gesture control mechanism (such as described below), or a consequence of the user engaging with or attempting to engage with the contact portion. For example, the adaptive learning algorithm may be trained with a learning routine where various people interact with the device, and provide information as to whether the interaction with the input device was intentional.

### 5.5 AIR CIRCUIT

An air circuit 4170 in accordance with an aspect of the present technology is a conduit or a tube constructed and arranged to allow, in use, a flow of air to travel between two components such as RPT device 4000 and the patient interface 3000 or 3800.

In particular, the air circuit 4170 may be in fluid connection with the outlet of the pneumatic block 4020 and the patient interface. The air circuit may be referred to as an air delivery tube. In some cases, there may be separate limbs of the circuit for inhalation and exhalation. In other cases, a single limb is used.

In some forms, the air circuit 4170 may comprise one or more heating elements configured to heat air in the air circuit, for example to maintain or raise the temperature of the air. The heating element may be in a form of a heated wire circuit, and may comprise one or more transducers, such as temperature sensors. In one form, the heated wire circuit may be helically wound around the axis of the air circuit 4170. The heating element may be in communication with a controller such as a central controller 4230. One example of an air circuit 4170 comprising a heated wire circuit is described in United States Patent 8733349.

### 5.5.1 Supplementary gas delivery

In one form of the present technology, supplementary gas, e.g. oxygen, 4180 is delivered to one or more points in the pneumatic path, such as upstream of the pneumatic block 4020, to the air circuit 4170, and/or to the patient interface 3000 or 3800.

### 5.6 HUMIDIFIER

### 5.6.1 Humidifier overview

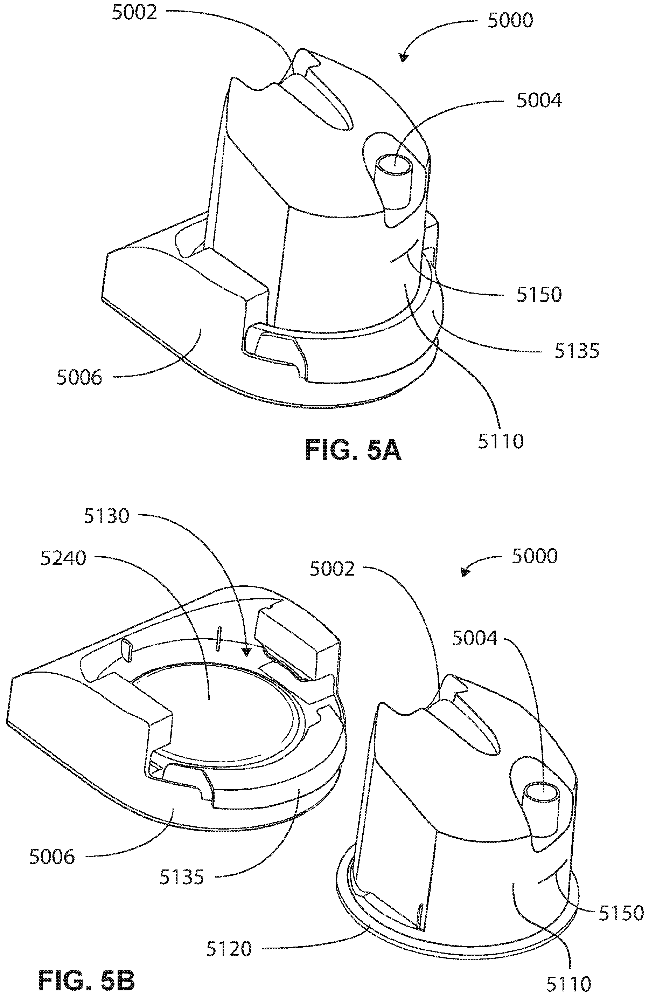

In one form of the present technology there is provided a humidifier 5000 (e.g. as shown in Fig. 5A) to change the absolute humidity of air or gas for delivery to a patient relative to ambient air. Typically, the humidifier 5000 is used to increase the absolute humidity and increase the temperature of the flow of air (relative to ambient air) before delivery to the patient's airways.

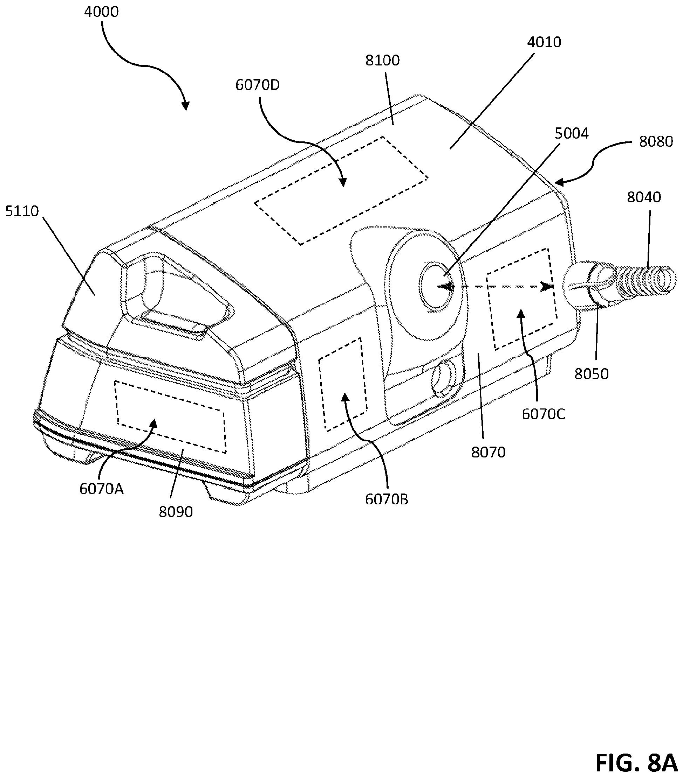

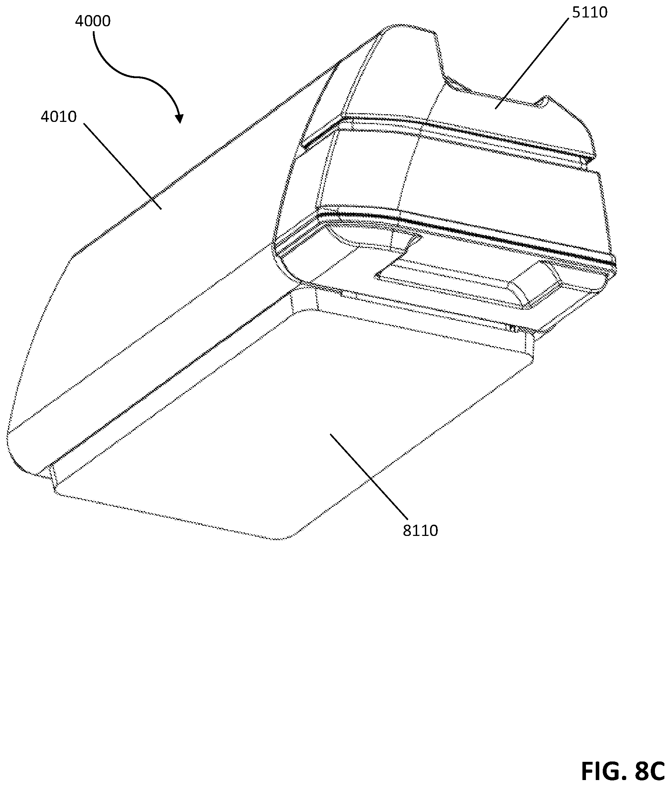

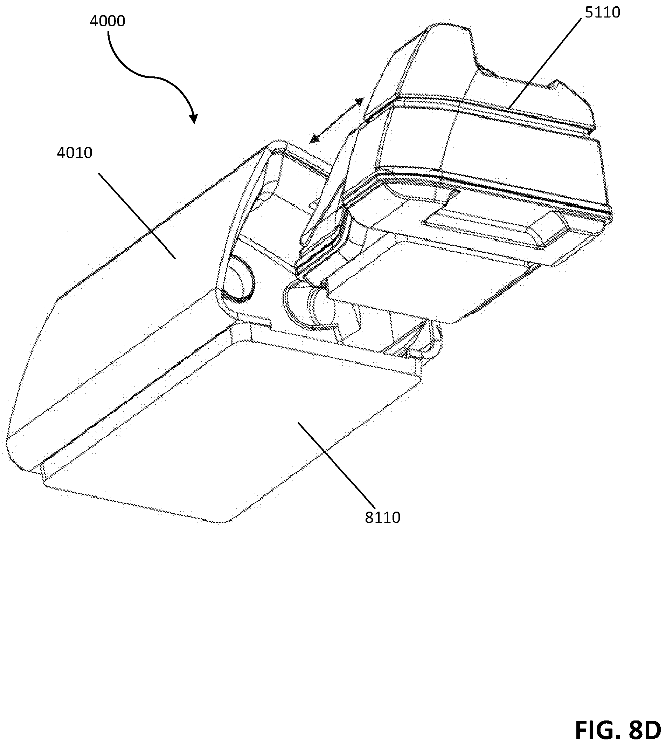

The respiratory therapy devices described herein may include an integral humidifier (as shown in Figures 8A to 8D). Alternatively, the humidifier may be a separate unit to the respiratory therapy device but fluidly connected thereto, for example by means of a fluid conduit. Alternatively, the humidifier and RPT device in some forms of the invention may be able to be assembled and disassembled where, in the assembled configuration they form a single device.

The humidifier 5000 may comprise a humidifier reservoir 5110, an inlet 5002 to receive a flow of air, and an outlet 5004 to deliver a flow of air which may be selectively humidified. In some forms, as shown in Fig. 5A and Fig. 5B, an inlet and an outlet of the humidifier reservoir 5110 may be the inlet 5002 and the outlet 5004 of the humidifier respectively. The humidifier 5000 may further comprise a humidifier base 5006, which may be adapted to receive the humidifier reservoir 5110 and comprise a heating element 5240.

The humidifier 5000 may comprise one or more components configured to detect whether the humidifier reservoir 5110 is correctly positioned or correctly attached to a respiratory therapy device. For example, said one or more components may be configured to ensure that the humidifier reservoir is positioned within the humidifier base 5006 or operatively (e.g. fluidly) connected to the respiratory therapy device. For example, the humidifier may include a switch which is closed when the humidifier reservoir 5110 is in position and open when the humidifier is removed. Alternatively, the humidifier 5000 or respiratory therapy device may detect the presence of the humidifier reservoir by using one or more wired or wireless connections to the humidifier reservoir 5110.

### 5.6.2 Humidifier components

### 5.6.2.1 Water reservoir

According to one arrangement, the humidifier 5000 may comprise a water reservoir 5110 configured to hold, or retain, a volume of liquid (e.g. water) to be evaporated for humidification of the flow of air. The water reservoir 5110 may be configured to hold a predetermined maximum volume of water in order to provide adequate humidification for at least the duration of a respiratory therapy session, such as one evening of sleep. Typically, the reservoir 5110 is configured to hold several hundred millilitres of water, e.g. 300 millilitres (ml), 325 ml, 350 ml or 400 ml. In other forms, the humidifier 5000 may be configured to receive a supply of water from an external water source such as a building's water supply system.

According to one aspect, the water reservoir 5110 is configured to add humidity to a flow of air from the RPT device 4000 as the flow of air travels therethrough. In one form, the water reservoir 5110 may be configured to encourage the flow of air to travel in a tortuous path through the reservoir 5110 while in contact with the volume of water therein.

According to one form, the reservoir 5110 may be removable from the humidifier 5000, for example in a lateral direction as shown in Fig. 5A and Fig. 5B.

The reservoir 5110 may also be configured to discourage egress of liquid therefrom, such as when the reservoir 5110 is displaced and/or rotated from its normal, working orientation, such as through any apertures and/or in between its subcomponents. As the flow of air to be humidified by the humidifier 5000 is typically pressurised, the reservoir 5110 may also be configured to prevent losses in pneumatic pressure through leak and/or flow impedance.

### 5.6.2.2 Conductive portion

According to one arrangement, the reservoir 5110 comprises a conductive portion 5120 configured to allow efficient transfer of heat from the heating element 5240 to the volume of liquid in the reservoir 5110. In one form, the conductive portion 5120 may be arranged as a plate, although other shapes may also be suitable. All or a part of the conductive portion 5120 may be made of a thermally conductive material such as aluminium (e.g. approximately 2 mm thick, such as 1 mm, 1.5 mm, 2.5 mm or 3 mm), another heat conducting metal or some plastics. In some cases, suitable heat conductivity may be achieved with less conductive materials of suitable geometry.

### 5.6.2.3 Humidifier reservoir dock

In one form, the humidifier 5000 may comprise a humidifier reservoir dock 5130 (as shown in Fig. 5B) configured to receive the humidifier reservoir 5110. In some arrangements, the humidifier reservoir dock 5130 may comprise a locking feature such as a locking lever 5135 configured to retain the reservoir 5110 in the humidifier reservoir dock 5130. The locking feature may further provide a means of detecting when the humidifier reservoir 5110 is correctly positioned in the humidifier reservoir dock 5130. For example, the locking feature may include or be configured to engage with a sensor or switch. When activated this sensor or switch may send a signal to a controller to indicate that the locking feature is in the correct position, with the humidifier reservoir 5110 in place.

### 5.6.2.4 Water level indicator

The humidifier reservoir 5110 may comprise a water level indicator 5150 as shown in Fig. 5A-5B. In some forms, the water level indicator 5150 may provide one or more indications to a user such as the patient 1000 or a care giver regarding a quantity of the volume of water in the humidifier reservoir 5110. The one or more indications provided by the water level indicator 5150 may include an indication of a maximum, predetermined volume of water, any portions thereof, such as 25%, 50% or 75% or volumes such as 200 ml, 300 ml or 400ml.

### 5.6.2.5 Humidifier transducer(s)

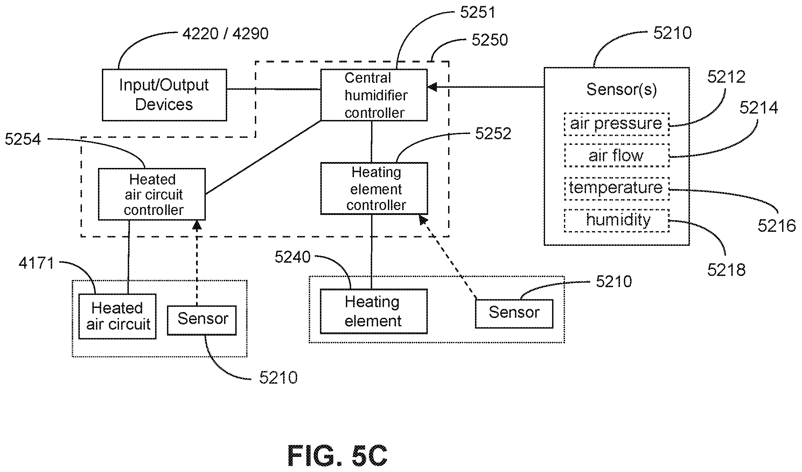

The humidifier 5000 may comprise one or more humidifier transducers (sensors) 5210 instead of, or in addition to, transducers 4270 described above. Humidifier transducers 5210 may include one or more of an air pressure sensor 5212, an *air* flow rate transducer 5214, a temperature sensor 5216, or a humidity sensor 5218 as shown in Fig. 5C. A humidifier transducer 5210 may produce one or more output signals which may be communicated to a controller such as the central controller 4230 and/or the humidifier controller 5250. In some forms, a humidifier transducer may be located externally to the humidifier 5000 (such as in the *air* circuit 4170) while communicating the output signal to the controller.

### 5.6.2.5.1 Pressure transducer

One or more pressure transducers 5212 may be provided to the humidifier 5000 in addition to, or instead of, a pressure sensor 4272 provided in the RPT device 4000.

### 5.6.2.5.2 Flow rate transducer

One or more flow rate transducers 5214 may be provided to the humidifier 5000 in addition to, or instead of, a flow rate sensor 4274 provided in the RPT device 4000.

### 5.6.2.5.3 Temperature transducer

The humidifier 5000 may comprise one or more temperature transducers 5216. The one or more temperature transducers 5216 may be configured to measure one or more temperatures such as of the heating element 5240 and/or of the flow of *air* downstream of the humidifier outlet 5004. In some forms, the humidifier 5000 may further comprise a temperature sensor 5216 to detect the temperature of the ambient air.

### 5.6.2.5.4 Humidity transducer

In one form, the humidifier 5000 may comprise one or more humidity sensors 5218 to detect a humidity of a gas, such as the ambient air. The humidity sensor 5218 may be placed towards the humidifier outlet 5004 in some forms to measure a humidity of the gas delivered from the humidifier 5000. The humidity sensor may be an absolute humidity sensor or a relative humidity sensor.

### 5.6.2.6 Heating element

A heating element 5240 may be provided to the humidifier 5000 in some cases to provide a heat input to one or more of the volume of water in the humidifier reservoir 5110 and/or to the flow of air. The heating element 5240 may comprise a heat generating component such as an electrically resistive heating track. One suitable example of a heating element 5240 is a layered heating element such as one described in the PCT Patent Application Publication No. WO2012/171072.

In some forms, the heating element 5240 may be provided in the humidifier base 5006 where heat may be provided to the humidifier reservoir 5110 primarily by conduction as shown in Fig. 5B.

### 5.6.2.7 Humidifier controller

According to one arrangement of the present technology, a humidifier 5000 may comprise a humidifier controller 5250 as shown in Fig. 5C. In one form, the humidifier controller 5250 may be a part of the central controller 4230. In another form, the humidifier controller 5250 may be a separate controller, which may be in communication with the central controller 4230.

In one form, the humidifier controller 5250 may receive as inputs measures of properties (such as temperature, humidity, pressure and/or flow rate), for example of the flow of air, the water in the reservoir 5110 and/or the humidifier 5000. The humidifier controller 5250 may also be configured to execute or implement humidifier algorithms and/or deliver one or more output signals.

As shown in Fig. 5C, the humidifier controller 5250 may comprise one or more controllers, such as a central humidifier controller 5251, a heated air circuit controller 5254 configured to control the temperature of a heated air circuit 4171 and/or a heating element controller 5252 configured to control the temperature of a heating element 5240.

### 5.7 USER DETECTION

### 5.7.1 Detection overview

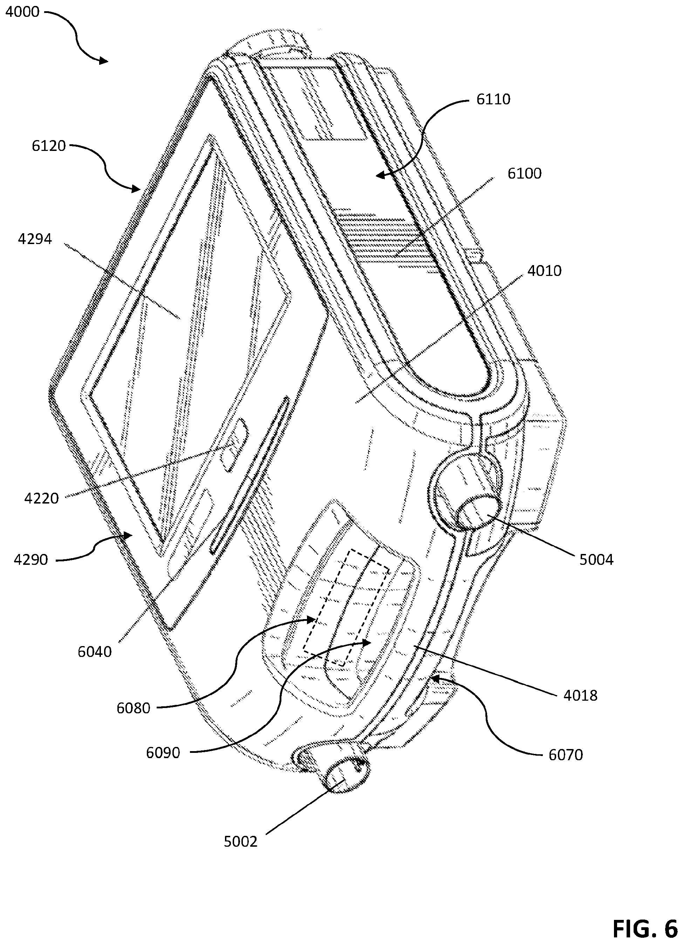

According to one form of the present technology a respiratory therapy device 4000 is provided. In the example of Fig. 6 the device 4000 includes an external housing 4010 which contains a pressure generator 4140 (not shown) for producing a flow of breathable gas at a positive pressure. For example, the pressure generator 4140 may be a controllable blower.

The device 4000 includes one or more input devices 4220 which in use allow the device to be configured and/or operated, for example by a user, for example a patient or physician. For example, the device 4000 includes input devices 4220 in the form of buttons. The device 4000 also includes output devices 4290 in the form of a display 4294, and an indicator light 6040 in the form of an LED light bar. The display 4294 in the illustrated form comprises a further input device 4220 in the form of a touch sensitive interface i.e. capacitive touch screen. In other forms of the technology the device 4000 may include a plurality of displays 4294 and/or a plurality of indicator lights 6040.

The device 4000 also includes inlet 5002 and outlet 5004 for directly or indirectly connecting to one or more peripheral devices such as conduits (heated or otherwise), patient interfaces, humidifiers, etc. In use the pressure generator supplies the flow of breathable gas to the outlet 5004.

The device 4000 also includes a contact portion 6070 in the form of a gripping portion or handle 4018 connected to the body of the RPT device 4000.

It should be understood that reference herein to contact portion(s) should be understood to include any feature or region of a device which is configured to be contacted by the user, either directly or indirectly to facilitate movement, carrying, lifting or use of the device. Indirect contact by the user may include, for example the user holding a tool that contacts the device, or wearing a garment that contacts the device.

In the illustrated form the handle 4018 is configured such that the part of the handle 4018 to be gripped by a user is separated from a body of the RPT device 4000 by an aperture 6090 which is configured to receive the fingers or hand of a user in use. However, this should not be seen as limiting on the technology, and in other forms the contact portion 6070 may be a region of the device which is textured or substantially smooth, the contact portion may also comprise one or more protrusions, knobs, ribs surfaces configured to facilitate user detection, motion detection, gesture detection or movement of the device. In other forms, the contact portion may comprise a recess in the external housing 4010 or a recess formed by a protrusion in the housing.

According to one form of the technology, the device 4000 includes one or more sensors (not shown) configured to detect the presence of a user in proximity to, the device 4000. For example, the user may be contacting the device 4000, or the contact portion 6070 thereof, or may be within the sensing range of the sensor. For example, the sensor may be a proximity sensor such as a capacitive, inductive, optical, radar, sonar, ultrasonic, or magnetic sensor. In other forms of the technology, the sensor may be a contact sensor. For example, the sensor may comprise a mechanical activation mechanism (such as a button), electrical contacts, capacitive, inductive or resistive touch technologies.

In Fig. 6, the sensor is located within the external housing 4010 of the device 4000. For example, the sensor may be located within the handle 4018 such that the sensor is arranged to detect the presence of a user's hand within the cavity or aperture 6090. In one form the sensor may be mounted on or adjacent to a back wall of the handle 4018 (where "back wall" in this context is intended to mean a wall of the handle 4018 immediately adjacent the aperture 6090 and facing towards the body of the RPT device), however this should not be seen as limiting on the technology, for example the sensor may be mounted on a side or front wall of the handle 4018 (the "front wall" in this context being the wall of the handle 4018 on the opposite side of the handle to the back wall and the side walls being in between the front and back walls). In other forms the sensor may be mounted within the handle 4018, with the sensing elements directed away from the cavity or aperture 6090. This arrangement may advantageously enable the sensor to better detect the user's hand prior to contact with the contact portion.

In other forms of the technology the sensor is positioned in a region 6080 of the housing 4010 which is adjacent to or opposite to the contact portion 6070, for example the region indicated by the dotted lines in Fig, 6. For example, a proximity sensor may be provided to the housing 4010 in region 6080. Use of a proximity sensor allows for detection of a user's hand near or on the contact portion 6070 without requiring the sensor to be directly mounted within or on the contact portion 6070. For example, the sensor could be used to detect a user's hand as it enters the cavity or aperture 6090 between the contact portion 6070 and the external housing 4010 of the device 4000.

In other forms of the technology at least part of the sensor may be mounted to an external or outer surface of the housing 4010. For example, the sensor may be a contact sensor provided to an external surface of the housing 4010. In one form of the technology, the contact sensor may be comprised as part of an adhesive label which is adhered to an outer surface of the housing 4010, such as on the handle 4018 of the device 4000. In other forms the contact sensor may comprise one or more contact surfaces which is configured to move relative to the housing 4010 when engaged by the user.

In forms of the technology, the device 4000 may include additional or alternative contact portions 6070 having different forms and/or positions to the contact portion 6070 shown in Fig. 6. For example, additional/alternative contact portion(s) 6070 may be provided to one or more sides 6110 or ends 6120 of the body of device 4000 to facilitate detection of a user attempting to move (e.g. lift) the device by holding the sides 6110.

### 5.7.2 Gesture Detection

In forms of the technology, the sensor may be configured to detect user movement, for example motion of the patient's body, specific gestures of a part of the user's body, or a sequence of motions or gestures. For example, a proximity sensor may be provided which is able to detect movement of the user relative to the device 4000.

In forms of the technology the sensor may be configured to detect a user moving towards the device 4000, away from the device 4000 or gesturing near the device 4000. For example, the sensor may be configured to detect a user gesturing near the device 4000, such as waving, moving their hand laterally or longitudinally across the device 4000 (or a contact portion 6070, or sensor of the device), in circular motions, or any other suitable gestures such as pinching, pointing or rotating their hands or fingers.

In forms of the technology where the device 4000 is configured to detect specific motions, the present technology may have greater immunity to false activations, and/or allow for a greater number of actions to be effected. In examples, the device 4000 may be configured to enable one or more output devices 4290 (for example a display) as a user approaches the device 4000 and disable one or more output devices 4290 as the user moves away from the device 4000. That is the device 4000 may be configured to effect a plurality of actions, where each action is effected in response to a different gesture. In other words, the device 4000 may be configured to distinguish between a plurality of motions or gestures by the user detected by the sensor, and effect a first action in response to detection of a first motion or gesture by the sensor and a second action in response to detection of a second motion or gesture by the sensor. In examples the first action may be different to the second action, however this should not be seen as limiting on the technology and in some forms the same action may be triggered by multiple different gestures.

Detection of motion or gestures may be used to effect one or more actions in the device 4000 such as increasing or decreasing volume, brightness, humidity, pressure or flow settings, dismissing notifications, silencing alerts or alarms, or changing operating modes of the device 4000 such as starting or stopping the delivery of breathable gas at a therapy pressure.

Use of gesture detection in a respiratory therapy device 4000 may advantageously enable users to interact with and/or control the device 4000 more easily. Particularly where the user is elderly, unwell or lacks the fine motor skills required to control traditional input devices 4220 such as buttons, dials and touchscreens.

In forms of the technology, the sensor may facilitate user engagement or interaction with the device 4000. This detection may be provided in one or more directions relative to the sensor or device 4000, such as in front of the device, above the device, behind the device, or proximate to one or more sides of the device. In forms of the technology, the device 4000 may comprise one or more markings or structures defining or indicating to the user a region proximate the device within which the user's movements are detected by the sensor. For example, the device may include one or more icons, contoured surfaces or protrusions on the outer surface of the housing which indicate to the user an approximate region proximate to the device where gestures may be detected by the sensor. These markings or structures may further provide a reference for the user to provide gestures relative to. For example, a user may gesture as if they are rotating a dial mounted within the marked region or on the structure in order to effect an action in the device 4000.

In forms of the technology, the sensor may comprise one or more processors configured to detect and/or distinguish between movements or gestures performed by the user. For example, the sensor may be configured to communicate specific gesture information (e.g. data representative of specific gestures) to the central controller 4230, which the central controller 4230 then uses to effect one or more actions as described herein. For example, the specific gesture information may include information that a swiping motion, stop gesture, or rotational gesture was detected. This specific gesture information may further comprise information relating to each gesture, such as the direction of movement, speed and or distance from the sensor. In this way the central controller 4230 may be able to determine whether an action should be effected in response to the gesture, for example if a gesture is detected at a distance which is beyond a pre-defined limit then the central controller 4230 may decide to not effect an action in response to the gesture.

In other forms of the technology, the sensor may communicate information regarding detected objects, such as distance, size, speed, direction of movement etc. to one or more processors or controllers (such as the central controller 4230). The processor or controller may then process the information in order to identify movement or gestures by a user. The information communicated from the sensor to the processor / controller may be data representative of movement in the sensor's field of view.

In a yet further form of the technology, the sensor may be configured to communicate substantially raw sensor data to a processor or controller, and the processor or controller may process this data to determine if a user is in proximity to the device or gesturing to the device. For example, the data may comprise data representative of distance of detected object(s) from the sensor, which the processor or controller processes in order to determine whether a user or gesture is detected and whether an action should be effected.

In certain forms of the technology, the processor that processes the data generated by the sensor (which may be central controller 4230, or another controller / processor, for example a processor forming part of the sensor itself) is configured to detect movements and/or gestures of the user, and optionally to distinguish between such movements and/or gestures, by applying one or more predetermined algorithms to the data, where the predetermined algorithms are configured to analyse the data in order to identify the appropriate movements and/or gestures.

In a yet further form of the technology, a controller or processor may be configured with a processing architecture that allows machine learning and/or artificial intelligence algorithms to be utilised in order to process one or more pieces of information from a sensor (such as raw data, object information, or gesture data). For example, a controller or processor may be configured with a processing architecture in the form of an artificial neural network which is pre-programmed or trained with a database of gesture information and/or is configured to undergo a learning routine in order to learn how to distinguish between data which is representative of a user gesturing and data which is not. The controller or processor may be configured to further learn during use of the device in order to account for variations in the way in which various users interact with or gesture to the device. Further adaptation of the processing architecture, for example the machine learning or artificial intelligence algorithms, may be automatic as the device is used, or updated in response to a specific learning routine. For example, the device may be configured to prompt a user to perform one or more prescribed gestures, sensed data from which the processor can apply to the processing architecture as reference gestures for reconfiguring the processing architecture and improving the accuracy of detection of any subsequent gestures that are detected. This prompting of the user may be performed during the initial set-up of the device, on a regular basis (such as 3-monthly), as required (for example when the device detects movement but is unable to determine any specific gestures) or when a learning routine is manually activated by the user (such as by selecting to activate the learning routine on the user interface).

In forms of the technology, the controller or processor may be configured to receive feedback on the accuracy of the gesture detection by analysis of data generated by sensing subsequent actions performed by the user. For example, the controller or processor may determine that a gesture to stop the flow of pressurised air is detected and effect an action accordingly (i.e. stop the flow of pressurised air). If subsequently the user reactivates the flow of pressurised air within a predetermined period of time (for example 10 seconds) then this may be interpreted by the controller or processor that that an incorrect gesture was identified or that an incorrect action was effected. In response to identifying one or more incorrect gestures or actions the device may be configured to prompt the user to perform a learning routine as described herein, or otherwise automatically update the device's behaviour to ignore or action similar gestures in a different manner in the future.

### 5.7.3 Actions

On detection of a user, the device 4000 may be configured to effect one or more actions either in the device 4000 itself or in a secondary device. For example, the device 4000 may be configured to effect one or more of the following actions:

- Disabling one or more features of an input device 4220. For example, the buttons, and/or touch-screen interface may be disabled to prevent inadvertent button presses as the device is being moved or transported;

- Disabling or enabling one or more features of the output devices 4290. For example, the display 4294 may be turned off to save power if a user is detected moving away from the device, or if no user is detected in proximity to the device. The device 4000 may further be configured to enable one or more features of the output device 4290, such as enabling the display, or a backlight of the display when a user is detected moving towards, or otherwise in proximity to the device 4000. Alternatively, or additionally the device 4000 may be configured to silence an alarm condition when a user is detected interacting or gesturing to the device 4000 or contact portion 6070 of the device 4000 i.e. the indicator light 6040 and/or any audible alarms may be silenced/disabled;

- Providing one or more messages, alerts or other feedback to the user indicating to the user that the device is active. For example, the user may be informed that the device 4000 should not be moved while in use. For example, the device may alert the user via one or more output device(s) of the device such as the display, an indicator light or a speaker. In other examples, the device may alert the user via one or more secondary, for example peripheral or remote devices. For example, the device may be configured to cause an audible, visual or tactile alert to be played via the user's cell phone. Such an action may be effected by the sensor sending a signal to a controller to indicate that the user has been detected, and the controller being configured to cause a transmitting device to send an actuation signal to a remote device such as a cell phone. In some forms the messages, alerts or other feedback may be provided through a combination of devices. In some forms the feedback may take the form of a message having text informing the user that the device should not be moved while in use. In other forms the feedback may take the form of an alert that implies this to the user, for example by illuminating a light associated with a warning message or symbol understood to be indicating not to move the device in use.

- Providing one or more messages, alerts or other feedback to the user concerning one or more releasable components connected to the device 4000. For example, the device may notify the user that a releasable component such as a conduit, or humidifier is connected to the inlet 5002 and/or outlet 5004 via one or more of the output devices 4290 of the device 4000 and/or a secondary device. The user may be notified through any one or more of visual, audible or tactile notification as indicated in the previous example;

- Providing one or more messages, alerts or other feedback to the user concerning any aspect of the use and/or operation of the RPT device 4000, humidifier 5000, and/or peripheral device; and

- Changing one or more operating parameters of the device 4000 or secondary device. For example, the device may determine that the user of the device 4000 is awake and lower the therapy pressure delivered by the device to improve user comfort. Alternatively, the user may provide one or more gestures to the device 4000 in order to effect one or more actions as described herein.

### 5.7.4 System for effecting actions

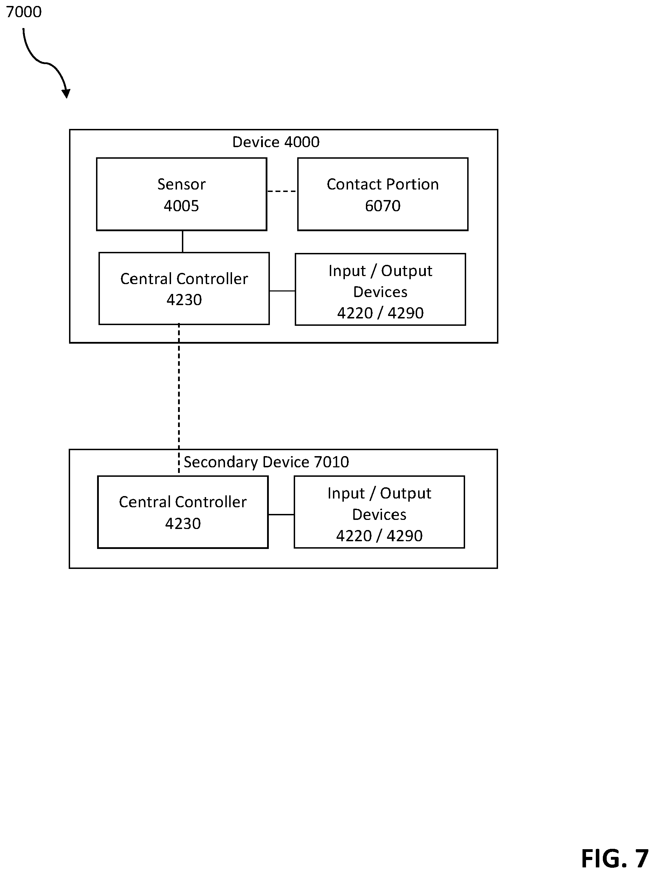

Fig. 7 is a block diagram illustrating a control system 7000 according to one form of the present technology. Control system 7000 is configured to effect one or more actions upon detection of a user in proximity to the device 4000.

An RPT device 4000 includes a sensor 4005 which is operatively connected to a controller, i.e. by a wired or wireless connection. For the sake of simplicity, reference herein will be made to the central controller 4230, however this should not be seen as limiting on the technology, and any suitable controller may be used. For example, a dedicated sensing controller may be used to determine the presence of an object, and/or distinguish between users and non-user objects such as walls in the manner described elsewhere in this specification. Central controller 4230 may comprise one or more discrete processing units.

In use, the sensor 4005, detects the presence of an object in proximity to the device, such as contacting the device or being proximate to the device 4000. For example, the sensor 4005 may be configured to detect objects within approximately 5 cm of a surface of the device 4000. In other forms of the technology it may be advantageous to configure the sensor 4005 to detect objects within approximately 1 cm of a surface of the device 4000. Limiting the range of the sensor 4005 may reduce the amount of data (such as information regarding nearby objects) the sensor or controller needs to process, potentially reducing the processing power required, and reducing the likelihood that a detection of an object other than a user could occur.

In further forms of the technology, it may be advantageous to configure the sensor 4005 to detect objects at a range which is greater than 5 cm from the device 4000. For example, the sensor 4005 may be configured to detect objects within approximately 2 metres of the device. Providing a sensing range greater than 5 cm may advantageously enable the device to detect a user as they approach or walk away from the device 4000.

In further forms of the technology, it may be advantageous to configure the sensor 4005 to detect objects between approximately 1 cm and 50 cm of the device 4000. Providing a range of between approximately 1 cm 50 cm may advantageously support gesture detection or motion detection without requiring the user to gesture too closely to the device, while reducing the likelihood that false activations could occur. It should be appreciated that the aforementioned ranges are provided by way of example only, and other suitable ranges may be used in accordance with the present technology.

In forms of the technology, the aforementioned distances and ranges may be measured relative to one or more contact portion(s) 6070 of the device 4000. For example, it may be advantageous to detect a user in proximity to a contact portion 6070 of the device 4000 in order to determine whether the user intends to move the device 4000.

The range and/or sensitivity of the sensor 4005 may also be adjustable. In one form the range of the sensor 4005 is manually adjustable i.e. adjustable by a technician or user. For example, the range may be adjusted using one of the input devices 4220 (such as a touch screen interface) or by manually adjusting a component of the sensor 4005 (such as a potentiometer). In another form, the range of the sensor is automatically adjustable, for example the sensor range may adjust based on the ambient conditions (i.e. time of day, temperature, moisture, ambient light levels etc). In other words, the central controller 4230 may monitor one or more ambient conditions, and in response to changes in the ambient conditions, send a signal to the sensor 4005 in order to adjust the sensitivity or range.

In forms of the technology, the sensor 4005 may be configured to continuously scan or monitor the surrounding environment for changes. For example, changes in the sensor's field or the amplitude of a signal reflected to the sensor may be indicative of a user approaching the device 4000, or contact portion(s) 6070 thereof. In other forms, the sensor 4005 may scan or monitor the surrounding environment on an intermittent basis. For example, once every second, or once every 5 seconds. Reducing the rate at which the sensor 4005 scans the surrounding environment may advantageously reduce the total power consumption of the device 4000. In yet further forms of the technology, the sensor 4005 may scan the surrounding environment at a variable rate. For example, the scan rate may be based on the number of proximate objects detected or the level or mode of use of the RPT device 4000. For example, the scan rate may be reduced when no proximate objects have been detected for a period of time, when the device is in use, or at night-time when users are less likely to be engaging with the device 4000.

In other forms, the sensor 4005 may be a contact sensor as described herein, i.e. the sensor may detect when a user touches the device 4000 or a contact portion 6070 thereof.

In use when the sensor 4005 and central controller 4230 determine that a user has touched, or is in proximity to, the device 4000 or contact portion 6070 thereof the controller may effect an action. For example, the controller may send a signal to one or both of the input device 4220 or output device 4290 to effect an action as described above. Alternatively, the controller may be configured to send a signal to a secondary device 7010 to effect an action in the secondary device 7010. For example, the central controller 4230 of the device 4000 may be configured to send a signal (either wired or wireless) to the central controller 4230 of the secondary device 5110. The central controller 4230 of the secondary device 7010 may then effect an action, for example, send a signal to one of the input 4220 or output devices 4290 of the secondary device 7010 to disable one or more features or to provide one or more alerts as described above.

## 5.7.5 Devices

Figs. 8A to 8D show a further respiratory therapy device 4000 according to another form of the present technology. The device includes an external housing 4010 which contains a pressure generator 4140 (not shown) for producing a flow of breathable gas at a positive pressure. For example, the pressure generator 4140 may be a controllable blower.

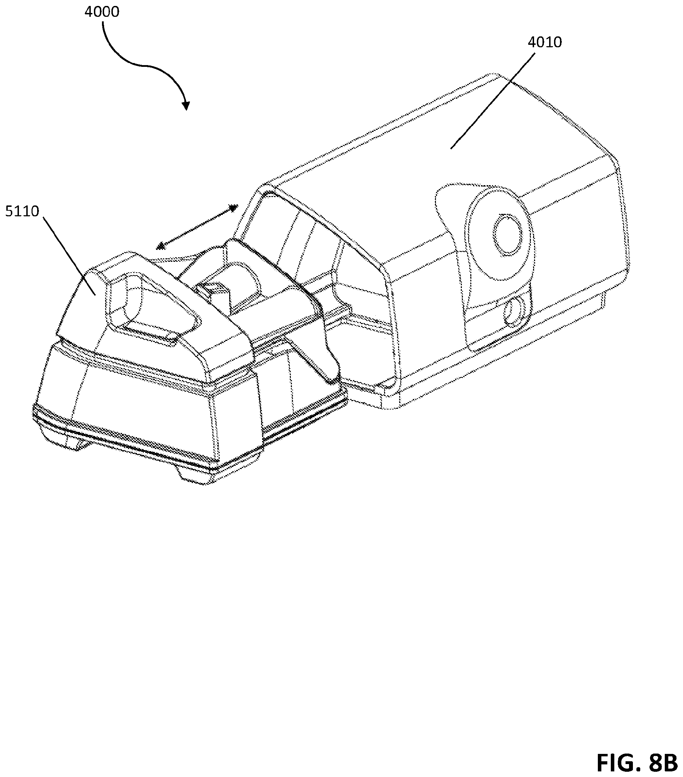

Releasably attached to the device is releasable component in the form of a water reservoir 5110 configured to hold a volume of water. For example, the water reservoir is slidably engaged with a cavity in the device 4000 as shown in Fig. 8B. The water reservoir 5110 may be used as part of a humidifier to humidify the flow of breathable gas for delivery to a patient's airways.

The device includes an outlet 5004 configured to receive a further releasable component in the form of a conduit 8040. The conduit 8040 is configured to facilitate delivery of the flow of breathable gas to the patient's airways in use. For example, the outlet 5004 may be configured to connect to a cuff 8050 of the conduit 8040 in use.

According to one form of the present technology the device 4000 is provided with at least one contact portion 6070. In one form the contact portion(s) 6070 are regions of the device 4000 which a user is likely to engage should they intend to move the device. These contact portions 6070 may include features which are likely to be touched by the user to effect movement of the device, such as protrusions, or textured surfaces. In other forms of the technology the contact portion 6070 may include recesses or cavities specifically designed to receive and/or accommodate part of the user, for example the user's hand, finger(s) and/or thumb, to facilitate movement of the device by the user.

For example, contact portions 6070 may be provided on or to one or more sides 8070 of the device, an end of the device 8080 (including an end 8090 of a releasable component such as the water reservoir 5110) or the top 8100 or bottom 8110 of the device. Exemplary contact portions 6070A, 6070B, 6070C, 6070D are illustrated in dashed lines in Fig. 8A.

In forms of the present technology where a contact portion 6070 is provided on or near to a water reservoir 5110, it may be advantageous for the device 4000 to alert the user via one or more output devices 4290 if a user is detected touching or approaching the water reservoir while the device 4000 is in use. For example, the controller may be configured to alert the user that the device 4000 should not be operational as the water reservoir is removed. Similarly, where contact portions are provided near to conduit or power cable connections, a similar warning may be provided via one or more output devices 4290.

The device 4000 includes one or more sensors (not shown) configured to detect the presence of a user making contact with or adjacent to the one or more contact portions 6070. The sensor may comprise any suitable proximity sensor or contact sensor as should be known to those skilled in the art.

Upon detection of a user the device 4000 may be configured to effect an action. For example, in addition to the actions described above, the device may provide feedback to the user concerning one or more releasable components connected to the device 4000. For example, the device may notify the user via one or more output devices that a releasable component such as the water reservoir 5110 or conduit 8040 is connected to the device 4000. This may advantageously reduce the likelihood of damage to the device when the device 4000 is moved.

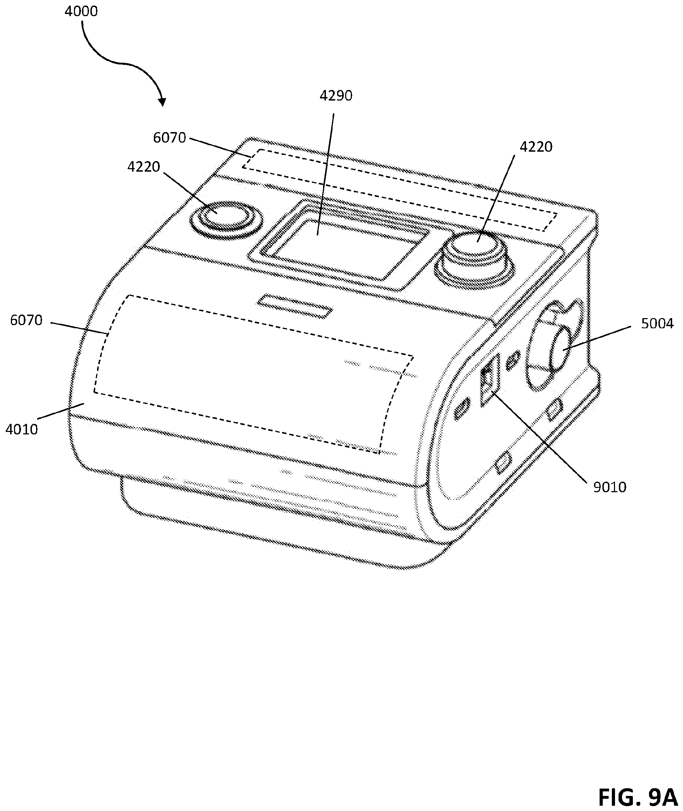

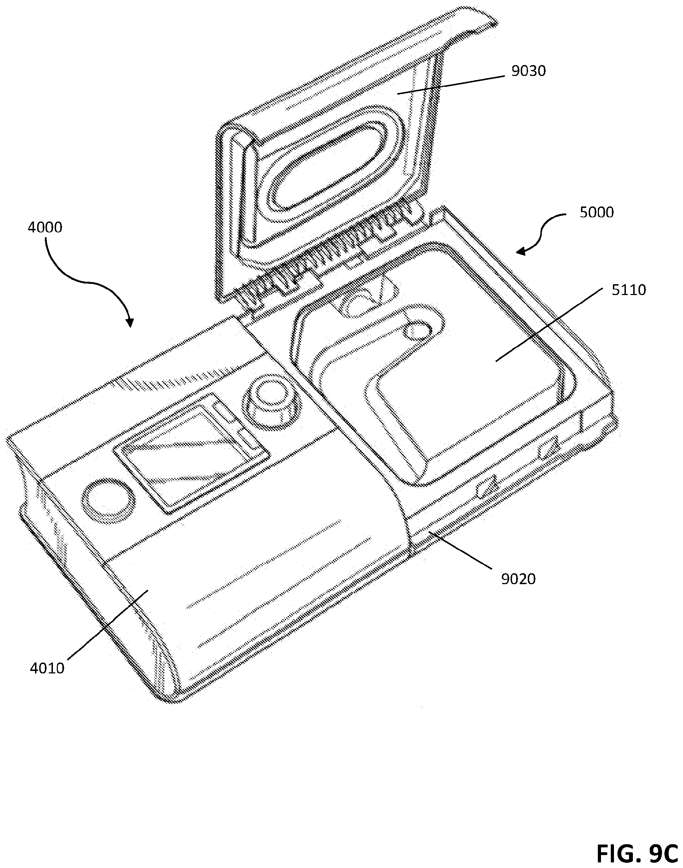

Figs. 9A to 9C show a further respiratory therapy device 4000 according to another form of the present technology. The device includes an external housing 4010 which contains a pressure generator 4140 (not shown) for producing a flow of breathable gas at a positive pressure. For example, the pressure generator 4140 may be a controllable blower.

The device 4000 also comprises input devices 4220 in the form of buttons, and dials, and output devices 4290 in the form of a display. The input devices 4220 and output devices 4290 together provide the user with a user interface for controlling and configuring the device 4000.

The device 4000 also comprises an outlet 5004 for supplying the flow of breathable gas to one or more downstream devices which are fluidly connected to the device 4000.

In one form of the technology, the device 4000 is configured to releasably connect to a releasable component in the form of humidifier 5000. The releasable connection between the device 4000 and the humidifier 5000 may be provided by a mechanical connection mechanism which, when engaged, aligns the device 4000 and humidifier 5000 in such a way that the outlet 5004 of the device 4000 is fluidly connected with an inlet of the humidifier 5000 and one or more electrical connections 9010 of the device 4000 are electrically connected to corresponding electrical connections on the humidifier 5000.

The humidifier has a humidifier housing 9020 which is separate to the external housing 4010 of the device 4000. The humidifier housing 9020 contains a water reservoir 5110 and a heating element 5240 (not shown) configured to heat the water contained in the water reservoir 5110 to thereby facilitate humidification of the flow of breathable gas.

The water reservoir 5110 may be removed from the humidifier housing 9020 by opening the lid or top cover 9030 of the humidifier 5000.

According to one form of the technology the device 4000 may comprise one or more contact portions 6070 configured to detect the presence of a hand or fingers of a user in use. For example, the device may include one or more contact portions 6070 as illustrated in the dashed regions of Fig. 9A. For example, the contact portions 6070 may be provided on a top surface, a front face, a side and/or back of the device 4000.

In use the device 4000 may be configured to effect an action upon detection of a user's hand or fingers in proximity to the device 4000 or one or more contact portions 6070 thereof as described herein. For example, the device 4000 may detect a user's hand on the housing of the device and thereby determine that the user is likely to be attempting to move the device 4000. In response the device 4000 may effect an action as has been previously described, for example the device 4000 may alert the user via one or more of the output devices that the humidifier 5000 is connected to the device 4000. For example, the device could display a warning on the screen of the device, or otherwise provide audible, visual or tactile feedback to the user.

Alerting the user that the device should not be moved while a releasable component is connected may advantageously protect the outlet 5004 and or electrical connections 9010 from damage which could occur if the device 4000 is moved while the releasable component is connected to it.

In a further form of the technology, the device 4000 may detect the presence of a user's hand or fingers in proximity to one or more contact portions 6070 and disable one or more input devices 4220 such as a button, dial or touch screen interface in order to prevent unintended operation of the device while the device is being transported.

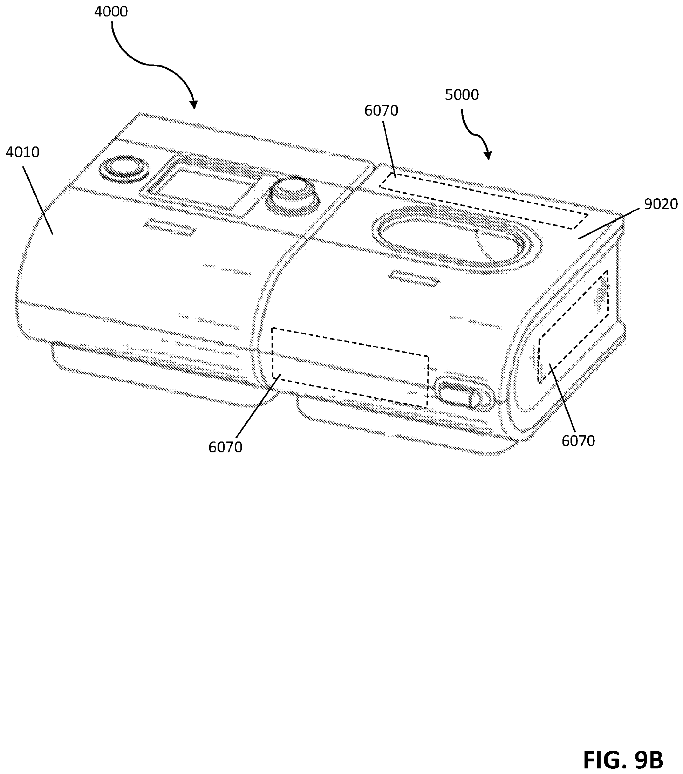

In a yet further form of the technology, one or more contact portions 6070 may be provided on the releasable component such as a humidifier 5000. For example, the humidifier 5000 may include one or more contact portions 6070 on a top surface, a front face, a side and/or back of the as illustrated in the dashed regions of Fig. 9B.

The contact portions 6070 shown in Fig. 9B comprise sensors configured to detect the presence of user's hand or fingers in accordance with the present technology. These sensors may be configured to communicate with a central controller 4230 housed within the device 4000 or the humidifier 5000. For example, a sensor positioned within the humidifier 5000 may be configured to communicate with the device 4000 by sending an electrical signal via the one or more electrical connections between the device 4000 and the humidifier 5000.

Positioning one or more sensors on a releasable component such as a humidifier may allow for the sensing of further types of interaction with the device or releasable component. For example, the device 4000 could detect when a user is engaging with a lid 9030 of the humidifier 5000, and may thereby determine that the user is attempting to refill or remove the water reservoir 5110. In response, the device 4000 or humidifier 5000 may effect an action such as temporarily disabling the heating element 5240 or flow of breathable gas. In humidifiers of the type having open-topped water reservoirs 5110, this may lessen the risk of heated, humidified gas being blown towards a user when the lid 9030 is opened.

In a further form of the technology, contact portions 6070 may be provided on both device 4000 and a releasable component such as humidifier 5000 in accordance with the present technology.

### 5.7.6 Contact Portions

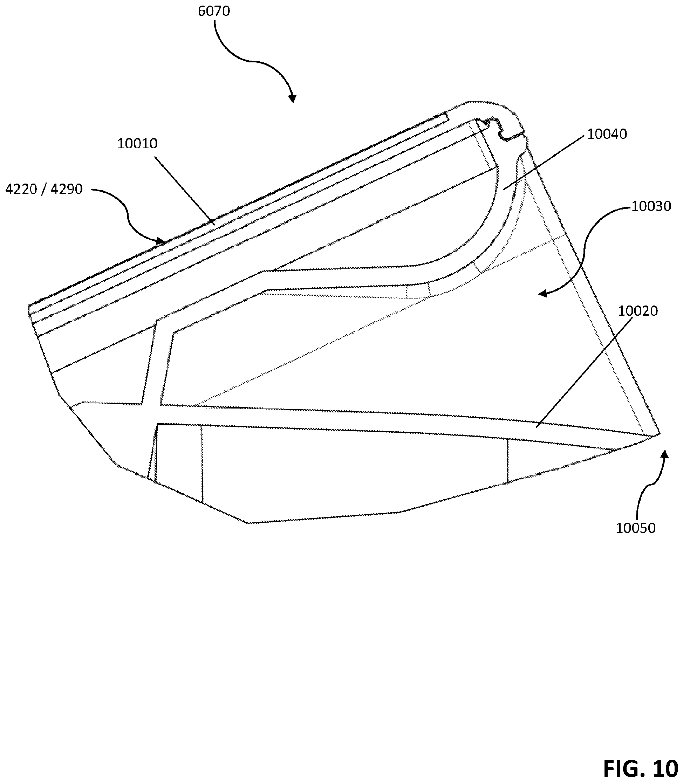

Fig. 10 shows a cross-sectional view of a contact portion 6070 of an RPT device 4000 according to one form of the present technology. In the illustrated form, the contact portion 6070 is formed from a protrusion 10010 extending outwardly from a top surface 10020 of the device 4000. In the form shown in Fig. 10 the protrusion takes the form of a plate. The plate extends outwardly from the body of the RPT device 4000 to form an overhang or cavity 10030 configured to receive a user's hand or fingers in use to facilitate lifting or movement of the device 4000. In the form illustrated, the protrusion 10010 extends from a frontwards portion of the RPT device 4000 towards the rear 10050 of the device 4000 at an acute angle when measured with reference to a horizontal plane when the device 4000 is resting on a horizontal surface. For example, the acute angle may be between approximately 10 degrees and approximately 40 degrees. In a preferred form the angle is between approximately 20 degrees and approximately 30 degrees. For example, the angle may be approximately 25 degrees. These angles may advantageously position the output device 4290 in a convenient orientation for a user to view and/or interact with one or more input 4220 and/or output devices 4290, such as displays, touch screen interfaces and buttons, provided to the upper surface of protrusion 10010 while providing a functional contact portion 6070 for the user to lift the device by inserting their hand or fingers under/into the overhang/cavity 10030 from a rearwards direction in relation to the intended orientation of the device during use.

The protrusion 10010 is further provided with a rib 10040 which a user can engage their fingers on to prevent the device 4000 slipping during movement of the RPT device 4000. In this way the rib 10040 provides a gripping portion for the user. In the form of the technology shown in Fig. 10 the rib 10040 is provided to an underside surface of protrusion 10010, for example to a distal part of the protrusion from the body of the RPT device 4000 to help form the cavity 10030 on a proximal side thereof.

The protrusion 10010 may also comprise, for example contain or have mounted thereto, a sensor for detecting the presence of the user's fingers or hand as described herein. For example, the sensor may be positioned within or on an underside of the rib 10040.

In other forms of the technology a sensor may be provided on the top surface 10020 of the housing 4010, underneath the protrusion 10010. In this way the sensor may be able to detect the presence of a user's hand within the cavity 10030 without needing to be installed within or attached to the protrusion 10010 (where space to mount the sensor may be limited).

In other forms of the technology, such as those illustrated in Fig. 8A to Fig. 9C the contact portion(s) 6070 may comprise one or more surface or regions on an outer surface of the device 4000. These surfaces or regions may be substantially clear of features such as protrusions, ribs or textured surfaces i.e. the contact portion(s) 6070 may comprise a clear region of the product which the user may contact or grip in use to lift or move the device.

In other examples, the contact portions may comprise one or more markings or structures which define or indicate to the user that the device provides a movement sensitive region which is proximate to the device. Where the sensor is configured to detect the user's movement within the movement sensitive region in order to effect an action in accordance with the present technology.

5.8 GLOSSARY

For the purposes of the present technology disclosure, in certain forms of the present technology, one or more of the following definitions may apply. In other forms of the present technology, alternative definitions may apply.

**5.8.1 General**

*Air*: In certain forms of the present technology, air may be taken to mean atmospheric air, and in other forms of the present technology air may be taken to mean some other combination of breathable gases, e.g. atmospheric air enriched with oxygen.

*Ambient*: In certain forms of the present technology, the term ambient will be taken to mean (i) external of the treatment system or patient, and (ii) immediately surrounding the treatment system or patient.

For example, ambient humidity with respect to a humidifier may be the humidity of air immediately surrounding the humidifier, e.g. the humidity in the room where a patient is sleeping. Such ambient humidity may be different to the humidity outside the room where a patient is sleeping.

In another example, ambient pressure may be the pressure immediately surrounding or external to the body.

In certain forms, ambient (e.g., acoustic) noise may be considered to be the background noise level in the room where a patient is located, other than for example, noise generated by an RPT device or emanating from a mask or patient interface. Ambient noise may be generated by sources outside the room.

*Automatic Positive Airway Pressure (APAP) therapy:* CPAP therapy in which the treatment pressure is automatically adjustable, e.g. from breath to breath, between minimum and maximum limits, depending on the presence or absence of indications of SDB events.

*Continuous Positive Airway Pressure (CPAP) therapy:* Respiratory pressure therapy in which the treatment pressure is approximately constant through a respiratory cycle of a patient. In some forms, the pressure at the entrance to the airways will be slightly higher during exhalation, and slightly lower during inhalation. In some forms, the pressure will vary between different respiratory cycles of the patient, for example, being increased in response to detection of indications of partial upper airway obstruction, and decreased in the absence of indications of partial upper airway obstruction.

*Flow rate:* The volume (or mass) of air delivered per unit time. Flow rate may refer to an instantaneous quantity. In some cases, a reference to flow rate will be a reference to a scalar quantity, namely a quantity having magnitude only. In other cases, a reference to flow rate will be a reference to a vector quantity, namely a quantity having both magnitude and direction. Flow rate may be given the symbol Q. 'Flow rate' is sometimes shortened to simply 'flow' or 'airflow'.

In the example of patient respiration, a flow rate may be nominally positive for the inspiratory portion of a breathing cycle of a patient, and hence negative for the expiratory portion of the breathing cycle of a patient. Device flow rate, $Qd$, is the flow rate of air leaving the RPT device. Total flow rate, $Qt$, is the flow rate of air and any supplementary gas reaching the patient interface via the air circuit. Vent flow rate, $Qv$, is the flow rate of air leaving a vent to allow washout of exhaled gases. Leak flow rate, $Ql$, is the flow rate of leak from a patient interface system or elsewhere. Respiratory flow rate, $Qr$, is the flow rate of air that is received into the patient's respiratory system.

*Flow therapy:* Respiratory therapy comprising the delivery of a flow of air to an entrance to the airways at a controlled flow rate referred to as the treatment flow rate that is typically positive throughout the patient's breathing cycle.

*Humidifier:* The word humidifier will be taken to mean a humidifying apparatus constructed and arranged, or configured with a physical structure to be capable of providing a therapeutically beneficial amount of water ($H_2O$) vapour to a flow of air to ameliorate a medical respiratory condition of a patient.

*Leak:* The word leak will be taken to be an unintended flow of air. In one example, leak may occur as the result of an incomplete seal between a mask and a patient's face. In another example leak may occur in a swivel elbow to the ambient.

*Noise, conducted (acoustic):* Conducted noise in the present document refers to noise which is carried to the patient by the pneumatic path, such as the air circuit and the patient interface as well as the air therein. In one form, conducted noise may be quantified by measuring sound pressure levels at the end of an air circuit.

*Noise, radiated (acoustic):* Radiated noise in the present document refers to noise which is carried to the patient by the ambient air. In one form, radiated noise may be quantified by measuring sound power/pressure levels of the object in question according to ISO 3744.

*Noise, vent (acoustic):* Vent noise in the present document refers to noise which is generated by the flow of air through any vents such as vent holes of the patient interface.

*Patient:* A person, whether or not they are suffering from a respiratory condition.

*Pressure:* Force per unit area. Pressure may be expressed in a range of units, including $cmH_2O$, g-f/cm$^2$ and hectopascal. 1 $cmH_2O$ is equal to 1 g-f/cm$^2$ and is approximately 0.98 hectopascal (1 hectopascal = 100 Pa = 100 N/m$^2$ = 1 millibar ~ 0.001 atm). In this specification, unless otherwise stated, pressure is given in units of $cmH_2O$.

The pressure in the patient interface is given the symbol $Pm$, while the treatment pressure, which represents a target value to be achieved by the interface pressure $Pm$ at the current instant of time, is given the symbol $Pt$.

*Respiratory Pressure Therapy (RPT):* The application of a supply of air to an entrance to the airways at a treatment pressure that is typically positive with respect to atmosphere.

*Ventilator:* A mechanical device that provides pressure support to a patient to perform some or all of the work of breathing.

### 5.8.1.1 Materials

*Silicone or Silicone Elastomer:* A synthetic rubber. In this specification, a reference to silicone is a reference to liquid silicone rubber (LSR) or a compression moulded silicone rubber (CMSR). One form of commercially available LSR is SILASTIC (included in the range of products sold under this trademark), manufactured by Dow Corning. Another manufacturer of LSR is Wacker. Unless otherwise specified to the contrary, an exemplary form of LSR has a Shore A (or Type A) indentation hardness in the range of about 35 to about 45 as measured using ASTM D2240. (Year? Required?)

*Polycarbonate:* a thermoplastic polymer of Bisphenol-A Carbonate.

### 5.8.1.2 Mechanical properties

*Resilience:* Ability of a material to absorb energy when deformed elastically and to release the energy upon unloading.

*Resilient:* Will release substantially all of the energy when unloaded. Includes e.g. certain silicones, and thermoplastic elastomers.

*Hardness:* The ability of a material *per se* to resist deformation (e.g. described by a Young's Modulus, or an indentation *hardness* scale measured on a standardised sample size).

- 'Soft' materials may include silicone or thermo-plastic elastomer (TPE), and may, e.g. readily deform under finger pressure.
- 'Hard' materials may include polycarbonate, polypropylene, steel or aluminium, and may not e.g. readily deform under finger pressure.

*Stiffness (or rigidity) of a structure or component:* The ability of the structure or component to resist deformation in response to an applied load. The load may be a force or a moment, e.g. compression, tension, bending or torsion. The structure or component may offer different resistances in different directions. The inverse of stiffness is *flexibility*.

*Floppy structure or component:* A structure or component that will change shape, e.g. bend, when caused to support its own weight, within a relatively short period of time such as 1 second.

*Rigid structure or component:* A structure or component that will not substantially change shape when subject to the loads typically encountered in use. An example of such a use may be setting up and maintaining a patient interface in sealing relationship with an entrance to a patient's airways, e.g. at a load of approximately 20 to 30 cmH$_2$O pressure.

As an example, an I-beam may comprise a different bending stiffness (resistance to a bending load) in a first direction in comparison to a second, orthogonal direction. In another example, a structure or component may be floppy in a first direction and rigid in a second direction.

## 5.8.2 Respiratory cycle

*Apnea:* According to some definitions, an apnea is said to have occurred when flow falls below a predetermined threshold for a duration, e.g. 10 seconds. An obstructive apnea will be said to have occurred when, despite patient effort, some obstruction of the airway does not allow air to flow. A central apnea will be said to have occurred when an apnea is detected that is due to a reduction in breathing effort, or the absence of breathing effort, despite the airway being patent. A mixed apnea occurs when a reduction or absence of breathing effort coincides with an obstructed airway.

*Breathing rate:* The rate of spontaneous respiration of a patient, usually measured in breaths per minute.

*Duty cycle:* The ratio of inhalation time, *Ti* to total breath time, *Ttot.*

*Effort (breathing):* The work done by a spontaneously breathing person attempting to breathe.

*Expiratory portion of a breathing cycle:* The period from the start of expiratory flow to the start of inspiratory flow.

*Flow limitation:* Flow limitation will be taken to be the state of affairs in a patient's respiration where an increase in effort by the patient does not give rise to a corresponding increase in flow. Where flow limitation occurs during an inspiratory portion of the breathing cycle it may be described as inspiratory flow limitation. Where flow limitation occurs during an expiratory portion of the breathing cycle it may be described as expiratory flow limitation.

Types of flow limited inspiratory waveforms:

*(i) Flattened:* Having a rise followed by a relatively flat portion, followed by a fall.

(ii) *M-shaped:* Having two local peaks, one at the leading edge, and one at the trailing edge, and a relatively flat portion between the two peaks.

(iii) *Chair-shaped:* Having a single local peak, the peak being at the leading edge, followed by a relatively flat portion.

(iv) *Reverse-chair shaped:* Having a relatively flat portion followed by single local peak, the peak being at the trailing edge.

*Hypopnea:* According to some definitions, a hypopnea is taken to be a reduction in flow, but not a cessation of flow. In one form, a hypopnea may be said to have occurred when there is a reduction in flow below a threshold rate for a duration. A central hypopnea will be said to have occurred when a hypopnea is detected that is due to a reduction in breathing effort. In one form in adults, either of the following may be regarded as being hypopneas:

(i) a 30% reduction in patient breathing for at least 10 seconds plus an associated 4% desaturation; or

(ii) a reduction in patient breathing (but less than 50%) for at least 10 seconds, with an associated desaturation of at least 3% or an arousal.

*Hyperpnea:* An increase in flow to a level higher than normal.

*Inspiratory portion of a breathing cycle:* The period from the start of inspiratory flow to the start of expiratory flow will be taken to be the inspiratory portion of a breathing cycle.

*Patency (airway):* The degree of the airway being open, or the extent to which the airway is open. A *patent* airway is open. Airway patency may be quantified, for example with a value of one (1) being patent, and a value of zero (0), being closed (obstructed).

*Positive End-Expiratory Pressure (PEEP):* The pressure above atmosphere in the lungs that exists at the end of expiration.

*Peak flow rate (Qpeak):* The maximum value of flow rate during the inspiratory portion of the respiratory flow waveform.

*Respiratory flow rate,* patient *airflow rate, respiratory airflow rate (Qr):* These terms may be understood to refer to the RPT device's estimate of respiratory flow rate, as opposed to "true respiratory flow rate" or "true respiratory flow rate", which is the actual respiratory flow rate experienced by the patient, usually expressed in litres per minute.

*Tidal volume (Vt):* The volume of air inhaled or exhaled during normal breathing, when extra effort is not applied. In principle the inspiratory volume *Vi* (the volume of air inhaled) is equal to the expiratory volume Ve (the volume of air exhaled), and therefore a single tidal volume *Vt* may be defined as equal to either quantity. In practice the tidal volume *Vt* is estimated as some combination, e.g. the mean, of the inspiratory volume *Vi* and the expiratory volume *Ve.*

*(inhalation) Time (Ti):* The duration of the inspiratory portion of the respiratory flow rate waveform.

*(exhalation) Time (Te):* The duration of the expiratory portion of the respiratory flow rate waveform.

*(total) Time (Ttot):* The total duration between the start of one inspiratory portion of a respiratory flow rate waveform and the start of the following inspiratory portion of the respiratory flow rate waveform.

*Typical recent ventilation:* The value of ventilation around which recent values of ventilation *Vent* over some predetermined timescale tend to cluster, that is, a measure of the central tendency of the recent values of ventilation.

*Upper airway obstruction (UAO):* includes both partial and total upper airway obstruction. This may be associated with a state of flow limitation, in which the flow rate increases only slightly or may even decrease as the pressure difference across the upper airway increases (Starling resistor behaviour).

*Ventilation (Vent):* A measure of a rate of gas being exchanged by the patient's respiratory system. Measures of ventilation may include one or both of inspiratory and expiratory flow, per unit time. When expressed as a volume per minute, this quantity is often referred to as "minute ventilation". Minute ventilation is sometimes given simply as a volume, understood to be the volume per minute.

### 5.8.3 Ventilation

*Adaptive Servo-Ventilator (ASV):* A servo-ventilator that has a changeable, rather than fixed target ventilation. The changeable target ventilation may be learned from some characteristic of the patient, for example, a respiratory characteristic of the patient.

*Backup rate:* A parameter of a ventilator that establishes the minimum breathing rate (typically in number of breaths per minute) that the ventilator will deliver to the patient, if not triggered by spontaneous respiratory effort.

*Cycled:* The termination of a ventilator's inspiratory phase. When a ventilator delivers a breath to a spontaneously breathing patient, at the end of the inspiratory portion of the breathing cycle, the ventilator is said to be cycled to stop delivering the breath.

*Expiratory positive airway pressure (EPAP):* a base pressure, to which a pressure varying within the breath is added to produce the desired interface pressure which the ventilator will attempt to achieve at a given time.

*End expiratory pressure (EEP):* Desired interface pressure which the ventilator will attempt to achieve at the end of the expiratory portion of the breath. If the pressure waveform template $\Pi(\Phi)$ is zero-valued at the end of expiration, i.e.

$$\Pi(\Phi) = 0 \text{ when } \Phi = 1, \text{ the EEP is equal to the EPAP.}$$

*Inspiratory positive airway pressure (IPAP):* Maximum desired interface pressure which the ventilator will attempt to achieve during the inspiratory portion of the breath.

*Pressure support:* A number that is indicative of the increase in pressure during ventilator inspiration over that during ventilator expiration, and generally means the difference in pressure between the maximum value during inspiration and the base pressure (e.g., *PS = IPAP - EPAP*). In some contexts, pressure support means the difference which the ventilator aims to achieve, rather than what it actually achieves.

*Servo-ventilator:* A ventilator that measures patient ventilation, has a target ventilation, and which adjusts the level of pressure support to bring the patient ventilation towards the target ventilation.

*Spontaneous/Timed (S/T):* A mode of a ventilator or other device that attempts to detect the initiation of a breath of a spontaneously breathing patient. If however, the device is unable to detect a breath within a predetermined period of time, the device will automatically initiate delivery of the breath.

*Swing:* Equivalent term to pressure support.

*Triggered:* When a ventilator delivers a breath of *air* to a spontaneously breathing patient, it is said to be triggered to do so at the initiation of the respiratory portion of the breathing cycle by the patient's efforts.

### 5.8.4 Anatomy

### 5.8.4.1 Anatomy of the face

*Ala:* the external outer wall or "wing" of each nostril (plural: alar)

*Alar angle:*

*Alare:* The most lateral point on the nasal *ala.*

*Alar curvature (or alar crest) point:* The most posterior point in the curved base line of each *ala,* found in the crease formed by the union of the *ala* with the cheek.

*Auricle:* The whole external visible part of the ear.

*(nose) Bony framework:* The bony framework of the nose comprises the nasal bones, the frontal process of the maxillae and the nasal part of the frontal bone.

*(nose) Cartilaginous framework:* The cartilaginous framework of the nose comprises the septal, lateral, major and minor cartilages.

*Columella:* the strip of skin that separates the nares and which runs from the pronasale to the upper lip.

*Columella angle:* The angle between the line drawn through the midpoint of the nostril aperture and a line drawn perpendicular to the Frankfort horizontal while intersecting subnasale.

*Frankfort horizontal plane:* A line extending from the most inferior point of the orbital margin to the left tragion. The tragion is the deepest point in the notch superior to the tragus of the auricle.

*Glabella:* Located on the soft tissue, the most prominent point in the midsagittal plane of the forehead.

*Lateral nasal cartilage:* A generally triangular plate of cartilage. Its superior margin is attached to the nasal bone and frontal process of the maxilla, and its inferior margin is connected to the greater alar cartilage.

*Lip, lower (labrale inferius)*:

*Lip, upper (labrale superius)*:

*Greater alar cartilage*: A plate of cartilage lying below the lateral nasal cartilage. It is curved around the anterior part of the naris. Its posterior end is connected to the frontal process of the maxilla by a tough fibrous membrane containing three or four minor cartilages of the *ala.*

*Nares (Nostrils):* Approximately ellipsoidal apertures forming the entrance to the nasal cavity. The singular form of nares is naris (nostril). The nares are separated by the nasal septum.

*Naso-labial sulcus or Naso-labial fold:* The skin fold or groove that runs from each side of the nose to the corners of the mouth, separating the cheeks from the upper lip.

*Naso-labial angle:* The angle between the columella and the upper lip, while intersecting subnasale.

*Otobasion inferior:* The lowest point of attachment of the auricle to the skin of the face.

*Otobasion superior*: The highest point of attachment of the auricle to the skin of the face.

*Pronasale:* the most protruded point or tip of the nose, which can be identified in lateral view of the rest of the portion of the head.

*Philtrum:* the midline groove that runs from lower border of the nasal septum to the top of the lip in the upper lip region.

*Pogonion:* Located on the soft tissue, the most anterior midpoint of the chin.

*Ridge (nasal):* The nasal ridge is the midline prominence of the nose, extending from the Sellion to the Pronasale.

*Sagittal plane:* A vertical plane that passes from anterior (front) to posterior (rear). The midsagittal plane is a sagittal plane that divides the body into right and left halves.

*Sellion:* Located on the soft tissue, the most concave point overlying the area of the frontonasal suture.

*Septal cartilage (nasal):* The nasal septal cartilage forms part of the septum and divides the front part of the nasal cavity.

*Subalare:* The point at the lower margin of the alar base, where the alar base joins with the skin of the superior (upper) lip.

*Subnasal point:* Located on the soft tissue, the point at which the columella merges with the upper lip in the midsagittal plane.

*Supramenton:* The point of greatest concavity in the midline of the lower lip between labrale inferius and soft tissue pogonion

### 5.8.4.2 Anatomy of the skull

*Frontal bone:* The frontal bone includes a large vertical portion, the *squama frontalis,* corresponding to the region known as the forehead.

*Mandible:* The mandible forms the lower jaw. The mental protuberance is the bony protuberance of the jaw that forms the chin.

*Maxilla:* The maxilla forms the upper jaw and is located above the mandible and below the orbits. The *frontal*

*process of the maxilla* projects upwards by the side of the nose, and forms part of its lateral boundary.

*Nasal bones:* The nasal bones are two small oblong bones, varying in size and form in different individuals; they are placed side by side at the middle and upper part of the face, and form, by their junction, the "bridge" of the nose.

*Nasion:* The intersection of the frontal bone and the two nasal bones, a depressed area directly between the eyes and superior to the bridge of the nose.

*Occipital bone:* The occipital bone is situated at the back and lower part of the cranium. It includes an oval aperture, the *foramen magnum,* through which the cranial cavity communicates with the vertebral canal. The curved plate behind the foramen magnum is the *squama occipitalis.*

*Orbit:* The bony cavity in the skull to contain the eyeball.

*Parietal bones:* The parietal bones are the bones that, when joined together, form the roof and sides of the cranium.

*Temporal bones:* The temporal bones are situated on the bases and sides of the skull, and support that part of the face known as the temple.

*Zygomatic bones:* The face includes two zygomatic bones, located in the upper and lateral parts of the face and forming the prominence of the cheek.

### 5.8.4.3 Anatomy of the respiratory system

*Diaphragm:* A sheet of muscle that extends across the bottom of the rib cage. The diaphragm separates the thoracic cavity, containing the heart, lungs and ribs, from the abdominal cavity. As the diaphragm contracts the volume of the thoracic cavity increases and air is drawn into the lungs.

*Larynx:* The larynx, or voice box houses the vocal folds and connects the inferior part of the pharynx (hypopharynx) with the trachea.

*Lungs:* The organs of respiration in humans. The conducting zone of the lungs contains the trachea, the bronchi, the bronchioles, and the terminal bronchioles. The respiratory zone contains the respiratory bronchioles, the alveolar ducts, and the alveoli.

*Nasal cavity:* The nasal cavity (or nasal fossa) is a large air filled space above and behind the nose in the middle of the face. The nasal cavity is divided in two by a vertical fin called the nasal septum. On the sides of the nasal cavity are three horizontal outgrowths called nasal conchae (singular "concha") or turbinates. To the front of the nasal cavity is the nose, while the back blends, via the choanae, into the nasopharynx.

*Pharynx:* The part of the throat situated immediately inferior to (below) the nasal cavity, and superior to the oesophagus and larynx. The pharynx is conventionally divided into three sections: the nasopharynx (epipharynx) (the nasal part of the pharynx), the oropharynx (mesopharynx) (the oral part of the pharynx), and the laryngopharynx (hypopharynx).

### 5.8.5 Patient interface

*Anti-asphyxia valve (AAV):* The component or sub-assembly of a mask system that, by opening to atmosphere in a failsafe manner, reduces the risk of excessive $CO_2$ rebreathing by a patient.

*Elbow:* An elbow is an example of a structure that directs an axis of flow of air travelling therethrough to change direction through an angle. In one form, the angle may be approximately 90 degrees. In another form, the angle may be more, or less than 90 degrees. The elbow may have an approximately circular cross-section. In another form the elbow may have an oval or a rectangular cross-section. In certain forms an elbow may be rotatable with respect to a mating component, e.g. about 360 degrees. In certain forms an elbow may be removable from a mating component, e.g. via a snap connection. In certain forms, an elbow may be assembled to a mating component via a one-time snap during manufacture, but not removable by a patient.

*Frame:* Frame will be taken to mean a mask structure that bears the load of tension between two or more points of connection with a headgear. A mask frame may be a non-airtight load bearing structure in the mask. However, some forms of mask frame may also be air-tight.

*Headgear:* Headgear will be taken to mean a form of positioning and stabilizing structure designed for use on a head. For example the headgear may comprise a collection of one or more struts, ties and stiffeners configured to locate and retain a patient interface in position on a patient's face for delivery of respiratory therapy. Some ties are formed of a soft, flexible, elastic material such as a laminated composite of foam and fabric.

*Membrane:* Membrane will be taken to mean a typically thin element that has, preferably, substantially no resistance to bending, but has resistance to being stretched.

*Plenum chamber:* a mask plenum chamber will be taken to mean a portion of a patient interface having walls at least partially enclosing a volume of space, the volume having air therein pressurised above atmospheric pressure in use. A shell may form part of the walls of a mask plenum chamber.

*Seal:* May be a noun form ("a seal") which refers to a structure, or a verb form ("to seal") which refers to the effect. Two elements may be constructed and/or arranged to 'seal' or to effect 'sealing' therebetween without requiring a separate 'seal' element *per se.*

*Shell:* A shell will be taken to mean a curved, relatively thin structure having bending, tensile and compressive stiffness. For example, a curved structural wall of a mask may be a shell. In some forms, a shell may be faceted. In some forms a shell may be airtight. In some forms a shell may not be airtight.

*Stiffener:* A stiffener will be taken to mean a structural component designed to increase the bending resistance of another component in at least one direction.

*Strut:* A strut will be taken to be a structural component designed to increase the compression resistance of another component in at least one direction.

*Swivel* (noun): A subassembly of components configured to rotate about a common axis, preferably independently, preferably under low torque. In one form, the swivel may be constructed to rotate through an angle of at least 360 degrees. In another form, the swivel may be constructed to rotate through an angle less than 360 degrees. When used in the context of an air delivery conduit, the sub-assembly of components preferably comprises a matched pair of cylindrical conduits. There may be little or no leak flow of air from the swivel in use.

*Tie* (noun): A structure designed to resist tension.

*Vent:* (noun): A structure that allows a flow of *air* from an interior of the mask, or conduit, to ambient air for clinically effective washout of exhaled gases. For example, a clinically effective washout may involve a flow rate of about 10 litres per minute to about 100 litres per minute, depending on the mask design and treatment pressure.

5.9 OTHER REMARKS

A portion of the disclosure of this patent document contains material which is subject to copyright protection. The copyright owner has no objection to the facsimile reproduction by anyone of the patent document or the patent disclosure, as it appears in Patent Office patent files or records, but otherwise reserves all copyright rights whatsoever.

Unless the context clearly dictates otherwise and where a range of values is provided, it is understood that each intervening value, to the tenth of the unit of the lower limit, between the upper and lower limit of that range, and any other stated or intervening value in that stated range is encompassed within the technology. The upper and lower limits of these intervening ranges, which may be independently included in the intervening ranges, are also encompassed within the technology, subject to any specifically excluded limit in the stated range. Where the stated range includes one or both of the limits, ranges excluding either or both of those included limits are also included in the technology.

Furthermore, where a value or values are stated herein as being implemented as part of the technology, it is understood that such values may be approximated, unless otherwise stated, and such values may be utilized to any suitable significant digit to the extent that a practical technical implementation may permit or require it.

Unless defined otherwise, all technical and scientific terms used herein have the same meaning as commonly understood by one of ordinary skill in the art to which this technology belongs. Although any methods and materials similar or equivalent to those described herein can also be used in the practice or testing of the present technology, a limited number of the exemplary methods and materials are described herein.

When a particular material is identified as being used to construct a component, obvious alternative materials with similar properties may be used as a substitute. Furthermore, unless specified to the contrary, any and all components herein described are understood to be capable of being manufactured and, as such, may be manufactured together or separately.

It must be noted that as used herein and in the appended claims, the singular forms "a", "an", and "the" include their plural equivalents, unless the context clearly dictates otherwise.

All publications disclose and describe the methods and/or materials which are the subject of those publications. The publications discussed herein are provided solely for their disclosure prior to the filing date of the present application. Nothing herein is to be construed as an admission that the present technology is not entitled to antedate such publication by virtue of prior invention. Further, the dates of publication provided may be different from the actual publication dates, which may need to be independently confirmed.

The terms "comprises" and "comprising" should be interpreted as referring to elements, components, or steps in a non-exclusive manner, indicating that the referenced elements, components, or steps may be present, or utilized, or combined with other elements, components, or steps that are not expressly referenced.

The subject headings used in the detailed description are included only for the ease of reference of the reader and should not be used to limit the subject matter found throughout the disclosure or the claims. The subject headings should not be used in construing the scope of the claims or the claim limitations.

Although the technology herein has been described with reference to particular examples, it is to be understood that these examples are merely illustrative of the principles and applications of the technology. In some instances, the terminology and symbols may imply specific details that are not required to practice the technology. For example, although the terms "first" and "second" may be used, unless otherwise specified, they are not intended to indicate any order but may be utilised to distinguish between distinct elements. Furthermore, although process steps in the methodologies may be described or illustrated in an order, such an ordering is not required. Those skilled in the art will recognize that such ordering may be modified and/or aspects thereof may be conducted concurrently or even synchronously.

It is therefore to be understood that numerous modifications may be made to the illustrative examples and that other arrangements may be devised without departing from the scope of the technology.

**Claims**

1. An apparatus for supplying a flow of breathable gas at a positive pressure for respiratory therapy, the apparatus comprising:

    a housing (4010) comprising an outlet (5004) and a contact portion (6070) that includes a gripping portion, the gripping portion being configured to be gripped by the user to facilitate movement or use of the apparatus,
    a pressure generator (4140) contained in the housing, the pressure generator for generating the flow of breathable gas and supplying the flow to the outlet; and
    a sensor (4005) configured to detect a user contacting or in proximity to the gripping portion, the sensor comprising one or more of a proximity sensor or a contact sensor,
    wherein, on detection by the sensor of the user contacting or being in proximity to the gripping portion, the apparatus is configured to effect an action.

2. The apparatus of claim 1, wherein the gripping portion comprises one or more of: a handle (4018), a cavity (6090, 10030) configured to receive at least a portion of the user's hand in use, and an outer surface of the housing (4010).

3. The apparatus of claim 1 or 2, wherein the action comprises disabling one or more of: an output device (4290), a display (4294), a backlight of a display, an input device (4220), a touch screen, and/or touch functionality of a touch screen.

4. The apparatus of any of claims 1 to 3, wherein on detection of the user, the apparatus is configured to alert the user of the presence or absence of a releasable component or that the apparatus is active.

5. The apparatus of claim 4, wherein the releasable component is a water reservoir (5110) or conduit (8040).

6. The apparatus of claim 4 or 5, wherein one or more contact portions (6070) are provided on the releasable component, the contact portions comprising sensors configured to detect the presence of a user's hand or fingers.

7. The apparatus of any one of claims 1 to 6, wherein the contact portion comprises a protrusion (10010).

8. The apparatus of claim 7, wherein the protrusion (10010) extends outwardly from a top surface of the housing (4010) at an acute angle.

9. The apparatus of claim 8, wherein the acute angle is between approximately 10 degrees and approximately 40 degrees, preferably between approximately 20 degrees and approximately 30 degrees.

10. The apparatus of any one of claims 7 to 9, wherein the protrusion (10010) provides an overhang or cavity (10030) configured to receive a user's hand in use.

11. The apparatus of any one of claims 7 to 10, wherein the protrusion (10010) includes an underside surface, and the gripping portion is provided as a rib (10040) on the underside surface.

12. The apparatus of claim 11, wherein the rib (10040) is provided on a distal part of the protrusion (10010) away from a body of the apparatus.

13. The apparatus of any one of claims 11 to 12, wherein the sensor is provided in the protrusion (10010), underneath the protrusion, within the rib (10040), or on the rib.

14. The apparatus of any one of claims 1 to 13, wherein the contact portion comprises one or more markings or structures defining or indicating to the user a region proximate the device within which the user's movement may be detected by the sensor.

15. The apparatus of any one of claims 1 to 14, wherein the proximity sensor comprises a capacitive, inductive, optical, radar, sonar, ultrasonic, or magnetic sensor, and the contact sensor comprises a button, electrical contacts, capacitive, inductive or piezo-electric resistive sensors.

**Patentansprüche**

1. Vorrichtung zum Zuführen eines Stroms von atembarem Gas mit einem Überdruck für eine Atemtherapie, wobei die Vorrichtung aufweist:

    ein Gehäuse (4010), das einen Auslass (5004) und einen Kontaktabschnitt (6070) aufweist, der einen Greifabschnitt aufweist, wobei der Greifabschnitt dazu konfiguriert ist, vom Benutzer ergriffen zu werden, um eine Bewegung oder Verwendung der Vorrichtung zu erleichtern,
    einen Druckgenerator (4140), der in dem Gehäuse enthalten ist, wobei der Druckgenerator dazu dient, den Strom von atembarem Gas zu erzeugen und den Strom dem Auslass zuzuführen; und
    einen Sensor (4005), der dazu konfiguriert ist, einen Benutzer zu erkennen, der den Greifabschnitt berührt oder sich in dessen Nähe befindet, wobei der Sensor einen oder mehrere Näherungssensoren oder Kontaktsensoren aufweist,
    wobei die Vorrichtung zum Durchführen einer Funktion konfiguriert ist, wenn der Sensor erkennt, dass der Benutzer den Greifabschnitt berührt oder sich in dessen Nähe befindet.

2. Vorrichtung nach Anspruch 1, wobei der Greifabschnitt eines oder mehrere der folgenden aufweist: einen Griff (4018), einen Hohlraum (6090, 10030), der im Gebrauch zum Aufnehmen mindestens eines Teils der Hand des Benutzers konfiguriert ist, und eine Außenfläche des Gehäuses (4010).

3. Vorrichtung nach Anspruch 1 oder 2, wobei die Funktion ein Deaktivieren eines oder mehrerer der folgenden umfasst: einer Ausgabevorrichtung (4290), einer Anzeige (4294), einer Hintergrundbeleuchtung einer Anzeige, einer Eingabevorrichtung (4220), eines Berührungsbildschirms und/oder einer Berührungsfunktionalität eines Berührungsbildschirms.

4. Vorrichtung nach einem der Ansprüche 1 bis 3, wobei die Vorrichtung dazu konfiguriert ist, bei Erkennen des Benutzers diesen auf ein Vorhandensein oder Nichtvorhandensein einer lösbaren Komponente oder darauf, dass die Vorrichtung aktiv ist, aufmerksam zu machen.

5. Vorrichtung nach Anspruch 4, wobei die lösbare Komponente ein Wasserreservoir (5110) oder eine Leitung (8040) ist.

6. Vorrichtung nach Anspruch 4 oder 5, wobei ein oder mehrere Kontaktabschnitte (6070) an der lösbaren Komponente vorgesehen sind, wobei die Kontaktabschnitte Sensoren aufweisen, die zum Erkennen der Anwesenheit einer Hand oder von Fingern des Benutzers konfiguriert sind.

7. Vorrichtung nach einem der Ansprüche 1 bis 6, wobei der Kontaktabschnitt einen Vorsprung (10010) aufweist.

8. Vorrichtung nach Anspruch 7, wobei sich der Vorsprung (10010) von einer Oberseite des Gehäuses (4010) in einem spitzen Winkel nach außen erstreckt.

9. Vorrichtung nach Anspruch 8, wobei der spitze Winkel zwischen etwa 10 Grad und etwa 40 Grad, bevorzugt zwischen etwa 20 Grad und etwa 30 Grad liegt.

10. Vorrichtung nach einem der Ansprüche 7 bis 9, wobei der Vorsprung (10010) einen Überhang oder Hohlraum (10030) bietet, der im Gebrauch zum Aufnehmen einer Hand eines Benutzers konfiguriert ist.

11. Vorrichtung nach einem der Ansprüche 7 bis 10, wobei der Vorsprung (10010) eine Unterseite aufweist und der Greifabschnitt als Rippe (10040) an der Unterseite vorgesehen ist.

12. Vorrichtung nach Anspruch 11, wobei die Rippe (10040) von einem Körper der Vorrichtung wegweisend an einem distalen Teil des Vorsprungs (10010) vorgesehen ist.

13. Vorrichtung nach einem der Ansprüche 11 bis 12, wobei der Sensor in dem Vorsprung (10010), unterhalb des Vorsprungs, innerhalb der Rippe (10040) oder auf der Rippe vorgesehen ist.

14. Vorrichtung nach einem der Ansprüche 1 bis 13, wobei der Kontaktabschnitt eine oder mehrere Markierungen oder Strukturen aufweist, die einen Bereich, innerhalb dessen die Bewegung des Benutzers durch den Sensor erfasst werden kann, in der Nähe der Vorrichtung definieren oder dem Benutzer angeben.

15. Vorrichtung nach einem der Ansprüche 1 bis 14, wobei der Näherungssensor einen kapazitiven, induktiven, optischen, Radar-, Sonar-, Ultraschall- oder magnetischen Sensor aufweist und der Kontaktsensor eine Taste, elektrische Kontakte, kapazitive, induktive oder piezoelektrische Widerstandssensoren aufweist.

## Revendications

1. Appareil pour fournir un flux de gaz respirable à une pression positive pour la thérapie respiratoire, l'appareil comprenant :

   un boîtier (4010) comprenant une sortie (5004) et une partie de contact (6070) qui comprend une partie de préhension, la partie de préhension étant configurée pour être saisie par l'utilisateur afin de faciliter le mouvement ou l'utilisation de l'appareil,
   un générateur de pression (4140) contenu dans le boîtier, le générateur de pression étant destiné à générer le flux de gaz respirable et à fournir le flux à la sortie ; et
   un capteur (4005) configuré pour détecter un utilisateur en contact ou à proximité de la partie de préhension, le capteur comprenant un ou plusieurs parmi des capteurs de proximité ou de contact,
   dans lequel, lors de la détection par le capteur de l'utilisateur en contact ou à proximité de la partie de préhension, l'appareil est configuré pour effectuer une action.

2. Appareil selon la revendication 1,
   dans lequel la partie de préhension comprend un ou plusieurs des éléments suivants : une poignée (4018), une cavité (6090, 10030) configurée pour recevoir au moins une partie de la main de l'utilisateur en cours d'utilisation, et une surface extérieure du boîtier (4010).

3. Appareil selon la revendication 1 ou 2,
   dans lequel l'action consiste à désactiver un ou plusieurs des éléments suivants : un dispositif de sortie (4290), un écran d'affichage (4294), un rétroéclairage d'un écran d'affichage, un dispositif d'entrée (4220), un écran tactile et/ou une fonctionnalité tactile d'un écran tactile.

4. Appareil selon l'une des revendications 1 à 3,
   dans lequel, lors de la détection de l'utilisateur, l'appareil est configuré pour alerter l'utilisateur de la présence ou de l'absence d'un composant amovible ou du fait que l'appareil est actif.

5. Appareil selon la revendication 4,
   dans lequel le composant amovible est un réservoir d'eau (5110) ou un conduit (8040).

6. Appareil selon la revendication 4 ou 5,
   dans lequel une ou plusieurs parties de contact (6070) sont prévues sur le composant amovible, les parties de contact comprenant des capteurs configurés pour détecter la présence de la main ou des doigts d'un utilisateur.

7. Appareil selon l'une des revendications 1 à 6,
   dans lequel la partie de contact comprend une protubérance (10010).

8. Appareil selon la revendication 7,
   dans lequel la protubérance (10010) s'étend vers l'extérieur à partir d'une surface supérieure du boîtier (4010) selon un angle aigu.

9. Appareil selon la revendication 8,

dans lequel l'angle aigu est compris entre environ 10 degrés et environ 40 degrés, de préférence entre environ 20 degrés et environ 30 degrés.

10. Appareil selon l'une des revendications 7 à 9,
dans lequel la protubérance (10010) constitue une saillie ou une cavité (10030) configurée pour recevoir la main de l'utilisateur en cours d'utilisation.

11. Appareil selon l'une des revendications 7 à 10,
dans lequel la protubérance (10010) comprend une surface inférieure, et la partie de préhension est prévue sous la forme d'une nervure (10040) à la surface inférieure.

12. Appareil selon la revendication 11,
dans lequel la nervure (10040) est prévue sur une partie distale de la protubérance (10010) éloignée du corps de l'appareil.

13. Appareil selon l'une des revendications 11 à 12,
dans lequel le capteur est prévue dans la protubérance (10010), en-dessous de la protubérance, à l'intérieur de la nervure (10040), ou sur la nervure.

14. Appareil selon l'une des revendications 1 à 13,
dans lequel la partie de contact comprend un ou plusieurs repères ou une ou plusieurs structures définissant ou indiquant à l'utilisateur une région proche du dispositif dans laquelle le mouvement de l'utilisateur peut être détecté par le capteur.

15. Appareil selon l'une des revendications 1 à 14,
dans lequel le capteur de proximité comprend un capteur capacitif, inductif, optique, radar, sonar, ultrasonique ou magnétique, et le capteur de contact comprend un bouton, des contacts électriques, des capteurs capacitifs, inductifs ou résistifs piézo-électriques.

FIG. 1A

EP 4 061 458 B1

**FIG. 1B**

**FIG. 2**

1000

3000

4000

5000

4170

FIG. 3

4015

4000

4018

4112

4015

4220

4012

Superior

Posterior

Anterior

Inferior

4202

4142

4100, 4020

4200

4010

4016

4210

4014

**FIG. 4A**

4110 — 4112 Air inlet filter ← Supplementary O₂

Pneumatic block

4120 — 4122 Inlet muffler

4270 — Transducer(s)

4140 — | 4142 Blower | 4144 Motor |

4180

4020

4120 — 4124 Outlet muffler

Upstream

4270 — Transducer(s)

Downstream

4160 — Anti-spillback valve

5000 — Humidifier

4110 — 4114 Filter

4180

4170 — Air circuit / delivery tube ← Supplementary O₂

4270 — Transducer(s)

4180

**FIG. 4B**

3000 — Patient Interface ← Supplementary O₂

FIG. 4C

**FIG. 5A**

**FIG. 5B**

FIG. 5C

FIG. 6

7000

Device 4000

| | |
|---|---|
| Sensor 4005 | Contact Portion 6070 |
| Central Controller 4230 | Input / Output Devices 4220 / 4290 |

Secondary Device 7010

| | |
|---|---|
| Central Controller 4230 | Input / Output Devices 4220 / 4290 |

FIG. 7

**FIG. 8A**

FIG. 8B

4000

5110

4010

8110

**FIG. 8C**

**FIG. 8D**

FIG. 9A

**FIG. 9B**

**FIG. 9C**

FIG. 10

**EP 4 061 458 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- AU 2019904413
- US 4944310 A, Sullivan
- US 6532959 B, Berthon-Jones
- US 20100000534
- WO 1998034665 A
- WO 2000078381 A
- US 6581594 B
- US 20090050156
- US 20090044808
- WO 2015089582 A
- US 7866944 B
- US 8638014 B
- US 8636479 B
- WO 2013020167 A
- US 8733349 B
- WO 2012171072 A

**Non-patent literature cited in the description**

- **JOHN B. WEST**. Respiratory Physiology. Lippincott Williams & Wilkins, 2012